# EUROPEAN PATENT APPLICATION

(11) **EP 4 231 018 A2**
(43) Date of publication of application: **23.08.2023**
(21) Application number: 23167412.8
(22) Date of filing: 07.07.2017
(51) Int. Cl.: G01N 33/74

(54) **ADRENOMEDULLIN FOR ASSESSING CONGESTION IN A SUBJECT WITH ACUTE HEART FAILURE**

(30) Priority: 08.07.2016 EP 16178725; 16.11.2016 EP 16199092
(62) Divisional of application: 17740693.1
(71) Applicant: SphingoTec GmbH, 16761 Henningsdorf (DE)
(72) Inventor: BERGMANN, Andreas, Berlin (DE); VOORS, Adriaan, Nietap (NL)
(74) Representative: Kilger, Ute

(57) **Abstract**

Subject matter of the present invention is a method for a) diagnosing congestion or assessing or monitoring the extent of congestion in a subject or, b) predicting or determining or monitoring need of therapy or intervention or predicting or determining or monitoring the success of a therapy or intervention of congestion or guiding a therapy or intervention in a subject or, c) predicting decongestion or residual congestion after therapy or intervention of congestion in a subject or, d) assessing decongestion or residual congestion after therapy or intervention of congestion in a subject or, e) assessing the decision on hospital discharge of a subject, wherein said subject is subject having acute heart failure that is either new-onset AHF or acute decompensated HF or acute decompensated chronic HF or wherein said subject is a subject having chronic heart failure with worsening signs/symptoms of chronic heart failure and wherein Pro-Adrenomedullin or fragments thereof of at least 5 amino acids is used as an early surrogate marker for congestion.

## Description

Subject matter of the present invention is a method for:
a) diagnosing congestion or assessing or monitoring the extent of congestion in a subject or,
b) predicting or determining or monitoring need of therapy or intervention of congestion or predicting or determining or monitoring the success of a therapy or intervention of congestion or guiding a therapy or intervention in a subject or,
c) predicting decongestion or residual congestion after therapy or intervention of congestion in a subject or,
d) assessing decongestion or residual congestion after therapy or intervention of congestion in a subject or,
e) assessing the decision on hospital discharge of a subject,
wherein said subject is a subject having acute heart failure that is either new-onset AHF or acute decompensated HF or acute decompensated chronic HF or wherein said subject is a subject having chronic heart failure with worsening signs/symptoms of chronic heart failure and wherein measurement of Pro-Adrenomedullin or fragments thereof of at least 5 amino acids is used as an early surrogate marker for congestion.

Heart failure (HF) is a cardiac condition that occurs, when a problem with the structure or function of the heart impairs its ability to supply sufficient blood flow to meet the body's needs. It can cause a large variety of symptoms, particularly shortness of breath (SOB) at rest or during exercise, signs of fluid retention such as pulmonary congestion or ankle swelling and objective evidence of an abnormality of the structure or function of the heart at rest.

Heart failure is a clinical syndrome characterized by a constellation of symptoms and signs caused by cardiac dysfunction. It is one of the major causes of morbidity and mortality in the developed countries, with a prevalence of 1-2%. Heart failure can be grouped into chronic HF and acute HF. Patients with chronic HF can be grouped into stable chronic HF, worsening signs and symptoms of chronic HF and acute decompensation of chronic HF. Acute heart failure (AHF) is defined as a rapid onset of signs and symptoms of heart failure resulting in the need for urgent therapy or hospitalization. AHF can present as acute de novo HF (new onset of AHF in a patient without previous cardiac dysfunction) or acute decompensation of chronic HF. AHF is the leading cause of hospitalization in adults older than 65 years of age. Despite marked improvements in the prognosis of chronic heart failure patients primarily related to therapeutic advances over the past few decades, both short- and long-term outcomes remain very poor once patients are hospitalized for decompensated heart failure. Nearly 25% of patients hospitalized for AHF need readmission within 30 days of hospital discharge while <50% survive beyond 5 years after hospitalization. In addition to significantly reducing survival and quality of life of affected patients, the monetary burden of AHF on health care systems is enormous. The total cost of heart failure care was estimated to be $31 billion in the US alone in 2012 and majority of this cost is associated with in-hospital care. This cost is projected to increase to an unprecedented $70 billion in 2030 due to ageing of populations.

Heart failure comprises a wide range of patients, from those with normal left ventricular ejection fraction (LVEF) typically considered as ≥50%, also known as HF with preserved EF (HFpEF) to those with reduced LVEF, typically considered as <40%, also known as HF with reduced EF (HFrEF). Patients with an LVEF in the range of 40-49% represent a `grey area', which is defined as HF with mid-range EF (HFmrEF) *(*Ponikowski et al. 2016. European Heart Journal 18(8): 891-975*).*

The main goal of AHF treatment in the in-hospital setting is decongestion (removal of excessive intra- and extracellular fluid simply put) and relief of symptoms and signs of congestion. Diuretics remain the main stay decongestive therapy in AHF and almost all hospitalized patients receive this class of drugs. Other classes of medications that increase cardiac output and reduce filling pressure; like inotropes and vasodilators are given in selected groups of patients. Ultrafiltration might also be considered in some patients, particularly in those who do not adequately respond to diuretic therapy.

Although patients (generally) respond well to diuretic therapy, a significant proportion of patients are discharged from hospital without attaining adequate level of decongestion and euvolemic status (i.e. residual congestion). This is primarily related to the fact that current approaches for the clinical assessment of congestion are inadequate. There is consistent evidence indicating that the presence of residual congestion during hospital discharge is associated with worse outcomes post-discharge, particularly hospital readmissions. Hence, there is a huge unmet need for a more accurate and reliable surrogate for congestion which can facilitate objective and optimal decision making regarding the adequacy of level of decongestion attained and timing of hospital discharge.

The peptide adrenomedullin (ADM) was described for the first time in Kitamura et al. *(*Kitamura et al. 1993. Biochemical and Biophysical Research Communications 192 (2): 553-560*)* as a novel hypotensive peptide comprising 52 amino acids, which had been isolated from a human pheochromocytoma. In the same year, cDNA coding for a precursor peptide comprising 185 amino acids and the complete amino acid sequence of this precursor peptide were also described. The precursor peptide, which comprises, inter alia, a signal sequence of 21 amino acids at the N-terminus, is referred to as "preproadrenomedullin" (pre-proADM). Pre-proADM comprises 185 amino acids. The mature ADM-NH₂ is displayed in SEQ ID No. 4 and ADM-Gly is displayed in SEQ No. 5.

The mature adrenomedullin peptide is an amidated peptide (ADM-NH₂), which comprises 52 amino acids (SEQ ID No: 4) and which comprises the amino acids 95 to 146 of pre-proADM, from which it is formed by proteolytic cleavage. To date, substantially only a few fragments of the peptide fragments formed in the cleavage of the pre-proADM have been more exactly characterized, in particular the physiologically active peptides adrenomedullin (ADM) and "PAMP", a peptide comprising 20 amino acids (22-41) which follows the 21 amino acids of the signal peptide in pre-proADM. Furthermore, for both, ADM and PAMP, physiologically active sub-fragments were discovered and investigated in more detail. The discovery and characterization of ADM in 1993 triggered intensive research activity and a flood of publications, the results of which have recently been summarized in various review articles, in the context of the present description, reference being made in particular to the articles Takahashi 2001. Peptides 22: 1691*;* Eto et al. 2001. Peptides 22: 1693-1711 *and* Hinson et al. 2000 Endocrine Reviews 21(2):138-167*.*

In the scientific investigations to date, it has been found, inter alia, that ADM may be regarded as a polyfunctional regulatory peptide. It is released into the circulation partially in an inactive form extended by glycine *(*Kitamura et al. 1998. Biochem. Biophys. Res. Commun. 244(2): 551-555*).* There is also a binding protein *(*Pio et al. 2001. The Journal of Biological Chemistry 276(15): 12292-12300*),* which is specific for ADM and probably likewise modulates the effect of ADM.

Those physiological effects of ADM as well as of PAMP, which are of primary importance in the investigations to date, were the effects influencing blood pressure. Thus, ADM is an effective vasodilator.

It has furthermore been found that the above-mentioned further physiologically active peptide PAMP formed from pre-proADM likewise exhibits a hypotensive effect, even if it appears to have an action mechanism differing from that of ADM *(*Eto et al. 2001. Peptides 22: 1693-1711*;* Hinson et al. 2000 Endocrine Reviews 21(2):138-167*;* Kuwasako et al. 1997. FEBS Lett 414(1): 105-110*;* Kuwasaki et al. 1999. Ann. Clin. Biochem. 36: 622-628*;* Tsuruda et al. 2001. Life Sci. 69(2): 239-245*:* Kangawa et al. EP 0 622 458*).*

Furthermore, it was found that the concentrations of ADM, which can be measured in the circulation and other biological fluids, are in a number of pathological states, significantly above the concentrations to be found in healthy control persons. Thus, the ADM level in patients with congestive heart failure, myocardial infarction, kidney diseases, hypertensive disorders, diabetes mellitus, in the acute phase of shock and in sepsis and septic shock are significantly increased, although to different extents. The PAMP concentrations are also increased in some of said pathological states, but the plasma levels are reduced relative to ADM *(*Eto et al. 2001. Peptides 22: 1693-1711*).*

Furthermore, it is known that unusual high concentrations of ADM are to be observed in sepsis or in septic shock *(*Eto et al. 2001. Peptides 22: 1693-1711*;* Hirata et al. 1996. Journal of Clinical Endocrinology and Metabolism 81(4): 1449-1453*;* Ehlenz et al.1997. Exp Clin Endocrinol Diabetes 105: 156-162*;* Tomoda et al. 2001. Peptides 22: 1783-1794*;* Ueda et al. 1999 Am. J. Respir. Crit. Care Med.160: 132-136*;* Wang et al. 2001. Peptides 22: 1835-1840*).* The findings are related to the typical hemodynamic changes which are known as typical phenomena of the course of a disease in patients with sepsis and other severe syndromes, such as, for example, SIRS. Adrenomedullin plays pivotal roles during sepsis development (Wang, Shock 1998, 10(5):383-384*;* Wang et al. 1998. Archives of surgery 133(12): 1298-1304*)* and in numerous acute and chronic diseases *(*Parlapiano et al. 1999. European Review for Medical and Pharmacological Sciences 3:53-61*;* Hinson et al. 2000 Endocrine Reviews 21(2):138-167*).*

Several methods were described to measure circulating levels of ADM: either ADM directly or indirectly by determining a more stable fragment of its cognate precursor peptide. Recently a method was published, describing an assay to measure circulating mature ADM *(*Marino et al. 2014. Crit Care 18: R34*).*

Other methods to quantify fragments derived from the ADM precursor have been described, e.g. the measurement of MR-proADM *(*Morgenthaler et al. 2005. Clin Chem 51(10):1823-9*),* PAMP *(*Washimine et al. 1994. Biochem Biophys Res Commun 202(2):1081-7*)* and CT-proADM *(*EP 2 111 552*).* A commercial homogeneous time-resolved fluoroimmunoassay for the measurement of MR-proADM in plasma on a fully automated system is available (BRAHMS MR-proADM KRYPTOR; BRAHMS GmbH, Hennigsdorf, Germany) *(*Caruhel et al. 2009. Clin Biochem 42(7-8):725-8*).* As these peptides are generated in a stoichiometric ratio from the same precursor, their plasma levels are correlated to a certain extent.

Plasma concentrations of ADM are elevated in patients with heart failure and correlate with disease severity (Hirayama et al. 1999. J Endocrinol 160: 297-303*;* Yu et al. 2001. Heart 86: 155-160)*.* High plasma ADM is an independent negative prognostic indicator in these subjects (Poyner et al. 2002. Pharmacol Rev 54: 233 246*).*

The role of MR-proADM in heart failure was explored in several studies. In the BACH study *(*Maisel et al. 2010. J. Am. Coll. Cardiol. 55: 2062 2076*),* MR-proADM was powerfully prognostic for death at 90 days, adding prognostic value beyond natriuretic peptides. Subsequent data from the PRIDE study *(*Shah et al. 2012. Eur. Heart J. 33: 2197-2205) solidified a potential prognostic role for MR-proADM; among patients MR-proADM had the best area under the curve (AUC) for mortality at 1 year. Similarly, levels of MR-proADM in patients with chronic heart failure (CHF) were strongly correlated with disease severity and elevated levels of the peptide were strongly associated with an increased risk of death at 12 months of follow-up (van Haehling et al. 2010. European Journal of Heart Failure 12: 484-491*;* Adlbrecht et al. 2009. European Journal of Heart Failure 11: 361-366*).*

MR-proADM was investigated during treatment in patients with acute decompensated heart failure *(*Boyer et al. 2012. Congest Heart Fail 18 (2): 91-97): patients whose MR-proADM levels tended to increase during acute therapy had findings associated with persistent congestion. In the 12-24 hour time-period after therapy, patients with elevations of MR-proADM had increased peripheral edema. Kaiser et al. measured MR-proADM in patients with univentricular hearts (Kaiser et al. 2014. Europ J Heart Failure 16: 1082-1088)*.* Levels in patients with a failed Fontan circuit (exhibiting ascites and peripheral edema) were significantly higher as compared to patients without Fontan failure. Moreover, Eisenhut speculated whether treatments leading to a reduction of adrenomedullin levels can reduce the severity and extent of alveolar edema in pneumonia and septicemia *(*Eisenhut 2006. Crit Care 10: 418*)*

Surprisingly, according to the present invention it has been found that Pro-Adrenomedullin or fragments thereof are an early and accurate surrogate for congestion in the setting of heart failure or acute heart failure, in particular in a subject having acute heart failure that is either new-onset AHF or acute decompensated HF or acute decompensated chronic HF or in a subject having chronic heart failure with worsening signs/symptoms of chronic heart failure.

Subject matter of the present invention is a method for:
a) diagnosing congestion or assessing or monitoring the extent of congestion in a subject or,
b) predicting or determining or monitoring need of therapy or intervention of congestion or predicting or determining or monitoring the success of a therapy or intervention of congestion or guiding a therapy or intervention of congestion in a subject or,
c) predicting decongestion or residual congestion after therapy or intervention of congestion in a subject or,
d) assessing decongestion or residual congestion after therapy or intervention of congestion in a subject or,
e) assessing the decision on hospital discharge of a subject,
wherein said subject is a subject having acute heart failure that is either new-onset AHF or acute decompensated HF or acute decompensated chronic HF or wherein said subject is a subject having chronic heart failure with worsening signs/symptoms of chronic heart failure and wherein Pro-Adrenomedullin or fragments thereof of at least 5 amino acids is used as an early surrogate marker for congestion comprising:
- Determining the level of Pro-Adrenomedullin or fragments thereof of at least 5 amino acids in a bodily fluid obtained from said subject; and

a) Correlating said level of Pro-Adrenomedullin or fragments thereof with the extent of congestion in said subject or diagnosing congestion, wherein an elevated level above a certain threshold is indicative of congestion or the extent of congestion or,
b) Correlating said level of Pro-Adrenomedullin or fragments thereof with the need or success of a therapy or intervention of congestion in said subject, wherein a level below a certain threshold is predictive for a success of therapy or intervention of congestion, and wherein a level above a certain threshold is indicative for the need of therapy or intervention of congestion, or
c) Correlating said level of Pro-Adrenomedullin or fragments thereof with a prediction of decongestion or residual congestion after therapy or intervention of congestion, wherein an elevated level above a certain threshold predicts residual congestion after therapy or intervention of congestion, whereas a level below a certain threshold predicts decongestion after therapy or intervention of congestion, or
d) Correlating said level of Pro-Adrenomedullin or fragments thereof with decongestion or residual congestion after therapy or intervention of congestion wherein an elevated level above a certain threshold indicates residual congestion after therapy or intervention of congestion whereas a level below a certain threshold indicates decongestion after therapy or intervention of congestion, or
e) Correlating said level of Pro-Adrenomedullin or fragments thereof with the assessment of the decision on hospital discharge wherein an elevated level above a certain threshold means that the subject shall not be discharged and wherein a level below a certain threshold means that the subject may be discharged,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH₂ according to SEQ ID No.: 4 or ADM-Gly according to SEQ ID No.: 5 or CT-proADM according to SEQ ID No.: 6.

Severity of congestion, extent of congestion, degree of congestion, grade of congestion and the like are used synonymously throughout this application.

Mature ADM, bio-ADMand ADM -NH₂ is used synonymously throughout this application and is a molecule according to SEQ ID No.: 4.

Therapy or intervention of congestion in a subject having acute heart failure and/or a subject having heart failure presenting with worsening signs and/or a subject with symptoms of heart failure or acute heart failure may be selected from the group comprising administration of diuretics, administration of inotropes, administration of vasodilators, ultrafiltration, in particular diuretics.

Pro-Adrenomedullin or fragments thereof are an early, quantitative and accurate surrogate for congestion in the setting of acute heart failure and heart failure, in particular in a subject having acute heart failure and/or a subject having heart failure presenting with worsening signs and/or a subject with symptoms of heart failure or acute heart failure. Early and accurate surrogate for congestion in the setting of acute heart failure or heart failure means that their concentration and/or level of immune reactivity reflects the extent of congestion, in particular the actual extent of congestion.

Throughout the specification the subject is a subject having acute heart failure and/or a subject having heart failure presenting with worsening signs and/or a subject with symptoms of heart failure or acute heart failure. In one particular aspect of the invention said subject has acute heart failure, that is either new-onset AHF or acute decompensated HF. In another particular aspect of the invention said subject has acute decompensated chronic HF or worsening signs/symptoms of chronic heart failure. In one particular aspect of the invention said subject has acute heart failure in particular new-onset AHF.

The term "acute" is used to mean rapid onset and to describe exacerbated or decompensated heart failure, referring to episodes in which a patient can be characterized as having a change in heart failure signs and symptoms resulting in a need for urgent therapy or hospitalization.

The term "chronic" refers to long duration. Chronic heart failure is a long-term condition, usually kept stable by the treatment of symptoms (stable chronic HF).

Stable chronic HF is characterized by:
(i) the presence of structural or functional failure of the heart that impairs its ability to supply sufficient blood flow to meet body's needs,
(ii) the absence of volume overload (manifested by pulmonary and/ or systemic congestion) and/ or profound depression of cardiac output (manifested by hypotension, renal insufficiency and/ or a shock syndrome),
and whereas the patient is not in need of urgent therapy or therapy adjustment and does not require hospitalization.

Chronic HF with worsening signs and symptoms is characterized by:
(i) the presence of structural or functional failure of the heart that impairs its ability to supply sufficient blood flow to meet body's needs,
(ii) volume overload (manifested by pulmonary and/ or systemic congestion) and/ or profound depression of cardiac output (manifested by hypotension, renal insufficiency and/ or a shock syndrome)
and whereas the patient is not in need of urgent therapy and does not require hospitalization, but is in need of therapy adjustment.

Chronic heart failure may also decompensate (termed acute decompensated heart failure or acute decompensated chronic heart failure), which is most commonly the result from an intercurrent illness (such as pneumonia), myocardial infarction, arrhythmias, uncontrolled hypertension or a patient's failure to maintain fluid restriction, diet or medication. After treatment, patients with acute decompensated chronic HF may return to a stable chronic compensated status (stable chronic HF).

New onset acute HF and acute decompensated chronic HF are characterized by:
(i) the presence of structural or functional failure of the heart that impairs its ability to supply sufficient blood flow to meet body's needs,
(ii) volume overload (manifested by pulmonary and/ or systemic congestion) and/ or profound depression of cardiac output (manifested by hypotension, renal insufficiency and/ or a shock syndrome)
and whereas the patient is in need of urgent therapy or therapy adjustment and does require hospitalization.

| **Acute HF** | | **Chronic HF** | | |
|---|---|---|---|---|
| New-onset AHF | Acute decompensated HF = acute decompensated chronic HF | | Worsening signs/ symptoms of chronic HF | Stable chronic HF |

The above definitions of acute heart failure that either new-onset AHF or acute decompensated HF or acute decompensated chronic HF or worsening signs/symptoms of chronic heart failure are in line with Voors et al., European Journal of Heart Failure (2016), 18, 716 - 726.

Congestion in HF is defined as a high left ventricular diastolic pressure associated with signs and symptoms of HF such as dyspnea, rales, and/or edema. These signs and symptoms related to congestion are the main reasons for HF-related hospitalizations.

Although relief of congestion (and associated signs/symptoms) and attainment of euvolemic status remain the main goal of inhospital AHF therapy, there is no standard algorithm or clinical tool for the assessment of congestion. Current practices regarding the clinical evaluation of congestion revolve around signs and symptoms. Physical examination findings such as elevated jugular venous pressure (JVP), peripheral edema, orthopnea, S3 heart sound and hepatomegaly or chest X-ray findings like cardiomegaly and interstitial/alveolar edema are used as surrogates for congestion. It must be noted that, besides a carefully performed JVP assessment, the predictive value of these parameters for detecting congestion is modest to moderate. There is a high unmet need to have a reliable and accurate surrogate marker for congestion. There is a high and unmet medical need to determining, predicting, assessing and/or monitoring congestion and decongestion in a quantitative and qualitative manner. There is the need to determining, predicting, assessing and/or monitoring the extent of congestion, i.e. the grade of congestion.

Thus, the methods of the present invention in all embodiments of the invention enable to
a) diagnosing quantitatively and qualitatively congestion in a subject and to assess or monitor the extent of congestion in a subject,
b) predicting or determining or monitoring need of therapy or intervention of congestion or predicting or determining or monitoring the success of a therapy or intervention of congestion or guiding a therapy or intervention in a subject or,
c) predicting decongestion or residual congestion after therapy or intervention of congestion in a subject or,
d) assessing decongestion or residual congestion after therapy or intervention of congestion in a subject or,
e) assessing the decision on hospital discharge of a subject,
wherein said subject is a subject having acute heart failure and/or a subject having heart failure presenting with worsening signs and/or a subject with symptoms of heart failure or acute heart failure by using Pro-Adrenomedullin or fragments thereof of at least 5 amino acids as an early surrogate marker for congestion as described in detail in the present invention.

In particular the methods of the present invention in all embodiments of the invention enable to diagnosing quantitatively and qualitatively congestion in a subject and to assess or monitor the extent of congestion in a subject, wherein said subject has acute heart failure that is either new-onset AHF or acute decompensated HF or acute decompensated chronic HF or wherein said subject has chronic heart failure with worsening signs/symptoms of chronic heart failure and wherein Pro-Adrenomedullin or fragments thereof is used of at least 5 amino acids as an early surrogate marker for congestion as described in detail in the present invention. In particular said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, preferably MR-proADM according to SEQ ID No.: 3 or mature ADM-NH₂ according to SEQ ID No.: 4 or ADM-Gly according to SEQ ID No.: 5, more preferably mature ADM-NH₂ according to SEQ ID No.: 4.

In particular the methods of the present invention in all embodiments of the invention enable to predicting or determining or monitoring need of therapy or intervention of congestion or predicting or determining or monitoring the success of a therapy or intervention of congestion or guiding a therapy or intervention in a subject wherein said subject has acute heart failure that is either new-onset AHF or acute decompensated HF or acute decompensated chronic HF or wherein said subject has chronic heart failure with worsening signs/symptoms of chronic heart failure and wherein Pro-Adrenomedullin or fragments thereof of at least 5 amino acids is used as an early surrogate marker for congestion as described in detail in the present invention. In particular said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, preferably MR-proADM according to SEQ ID No.: 3 or mature ADM-NH₂ according to SEQ ID No.: 4 or ADM-Gly according to SEQ ID No.: 5, more preferably mature ADM-NH₂ according to SEQ ID No.: 4.

In particular the methods of the present invention in all embodiments of the invention enable to predicting decongestion or residual congestion after therapy or intervention of congestion in a subject wherein said subject has acute heart failure that is either new-onset AHF or acute decompensated HF or acute decompensated chronic HF or wherein said subject has chronic heart failure with worsening signs/symptoms of chronic heart failure and wherein Pro-Adrenomedullin or fragments thereof of at least 5 amino acids is used as an early surrogate marker for congestion as described in detail in the present invention. In particular said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, preferably MR-proADM according to SEQ ID No.: 3 or mature ADM-NH₂ according to SEQ ID No.: 4 or ADM-Gly according to SEQ ID No.: 5, more preferably mature ADM-NH₂ according to SEQ ID No.: 4.

In particular the methods of the present invention in all embodiments of the invention enable to assessing decongestion or residual congestion after therapy or intervention of congestion in a subject wherein said subject has acute heart failure that is either new-onset AHF or acute decompensated HF or acute decompensated chronic HF or wherein said subject has chronic heart failure with worsening signs/symptoms of chronic heart failure and wherein Pro-Adrenomedullin or fragments thereof of at least 5 amino acids is used as an early surrogate marker for congestion as described in detail in the present invention. In particular said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, preferably MR-proADM according to SEQ ID No.: 3 or mature ADM-NH₂ according to SEQ ID No.: 4 or ADM-Gly according to SEQ ID No.: 5, more preferably mature ADM-NH₂ according to SEQ ID No.: 4.

In particular the methods of the present invention in all embodiments of the invention enable to assessing the decision on hospital discharge of a subject wherein said subject has acute heart failure that is either new-onset AHF or acute decompensated HF or acute decompensated chronic HF or wherein said subject has chronic heart failure with worsening signs/symptoms of chronic heart failure and wherein Pro-Adrenomedullin or fragments thereof of at least 5 amino acids is used as an early surrogate marker for congestion as described in detail in the present invention. In particular said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, preferably MR-proADM according to SEQ ID No.: 3 or mature ADM-NH₂ according to SEQ ID No.: 4 or ADM-Gly according to SEQ ID No.: 5, more preferably mature ADM-NH₂ according to SEQ ID No.: 4.

For the present invention the extent of congestion may be expressed as grade severity of congestion and has been determined as described below. However, the person skilled in the art knows that the extent of congestion may be expressed by other scores or surrogates, such as for instance the score utilized by Ambrosy et al. (Ambrosy et al. 2013. European Heart Journal 34 (11): 835-843)*.*

As highlighted previously, clinical surrogates have less than optimal predictive value for the detection of congestion. In the analysis for one of the examples shown in the present invention (PROTECT study), we combined three of the strongest clinical surrogates of congestion (i.e. JVP, peripheral edema and orthopnea) to enhance accuracy and developed a composite clinical congestion score (CCS) using the scheme presented below:

| Parameter | 0 | 1 | 2 | 3 |
|---|---|---|---|---|
| Peripheral edema | 0 | Ankle | Below knee | Above knee |
| Orthopnea | 0 pillow | 1 pillow | 2 pillows | 3 pillows |
| JVP | <6cm | 6-10cm | >10cm | - |

The score on each of these three parameters were then added to obtain a composite congestion score, which ranged from 0 to 8.

The following algorithm was then utilized to grade severity of congestion:
CCS=0, No clinical congestion
CCS 1-3, Mild clinical congestion
CCS 4-5, Moderate clinical congestion
CCS ≥6, Severe clinical congestion

In the analysis for another example shown in the present invention (BIOSTAT study), we described the grade of congestion by the level of peripheral edema (in the order of increasing severity: none, ankle, below knee, above knee). While PROTECT is a selected population of patients throughout the world with acute decompensated heart failure, enrolled in a randomized controlled clinical trial on the effects of rolofylline versus placebo, BIOSTAT-CHF consists of 2 European cohorts of patients with signs and/or symptoms of worsening heart failure, approximately 2/3 was hospitalized and 1/3 were seen in the outpatient setting.

As above explained, congestion can be classified in many different ways. The person skilled in the art knows that the extent of congestion may be expressed by other scores or surrogates. Clinical classification can be based on bedside physical examination in order to detect the presence of clinical symptoms/signs of congestion ('wet' vs. 'dry' if present vs. absent) and/or peripheral hypoperfusion ('cold' vs. 'warm' if present vs. absent) (for review see Ponikowski et al. 2016. Eur Heart J. ehw 128)*.* The combination of these options identifies four groups: warm and wet (well perfused and congested) - most commonly present; cold and wet (hypoperfused and congested); cold and dry (hypoperfused without congestion); and warm and dry (compensated, well perfused without congestion). This classification may be helpful to guide therapy in the initial phase and carries prognostic information.

Typically, symptoms and signs of AHF reflect fluid overload (pulmonary congestion and/or peripheral edema) or, less often, reduced cardiac output with peripheral hypoperfusion. Chest X-ray can be a useful test for the diagnosis of AHF. Pulmonary venous congestion, pleural effusion, interstitial or alveolar edema and cardiomegaly are the most specific findings for AHF, although in up to 20% of patients with AHF, chest X-ray is nearly normal.

Symptoms/signs of congestion (left-sided) are defined as orthopnoea, paroxysmal nocturnal dyspnoea, pulmonary rales (bilateral), peripheral edema (bilateral). Symptoms/signs of congestion (right-sided) are defined as jugular venous dilatation, peripheral edema (bilateral), congested hepatomegaly, hepatojugular reflux, ascites, symptoms of gut congestion (for review see table 12.2 in Ponikowski et al. 2016. Eur Heart J. ehw 128)*.*

Edema is an accumulation of fluid in the intercellular tissue that results from an abnormal expansion in interstitial fluid volume. The fluid between the interstitial and intravascular spaces is regulated by the capillary hydrostatic pressure gradient and the oncotic pressure gradient across the capillary *(*Traves et al. 2013. Am Fam Physician 88(2):102-110)*.* The accumulation of fluid occurs when local or systemic conditions disrupt this equilibrium, leading to increased capillary hydrostatic pressure, increased plasma volume, decreased plasma oncotic pressure (hypoalbuminemia), increased capillary permeability, or lymphatic obstruction.

Clinically, edema manifests as swelling: the amount of interstitial fluid is determined by the balance of fluid homeostasis, and the increased secretion of fluid into the interstitium, or the impaired removal of the fluid can cause edema. A rise in hydrostatic pressure occurs in cardiac failure. Causes of edema which are generalized to the whole body can cause edema in multiple organs and peripherally. For example, severe heart failure can cause pulmonary edema, pleural effusions, ascites and peripheral edema.

Pulmonary edema is fluid accumulation in the air spaces and parenchyma of the lungs. It leads to impaired gas exchange and may cause respiratory failure. It is due to either failure of the left ventricle of the heart to adequately remove blood from the pulmonary circulation ("cardiogenic pulmonary edema"), or an injury to the lung parenchyma or vasculature of the lung ("noncardiogenic pulmonary edema") *(*Ware and Matthay 2005. N. Engl. J. Med. 353 (26): 2788-96). Treatment is focused on three aspects: firstly improving respiratory function, secondly, treating the underlying cause, and thirdly avoiding further damage to the lung. Pulmonary edema, especially acute, can lead to fatal respiratory distress or cardiac arrest due to hypoxia. It is a cardinal feature of congestive heart failure.

The overwhelming symptom of pulmonary edema is difficulty breathing, but may also include coughing up blood (classically seen as pink, frothy sputum), excessive sweating, anxiety, and pale skin. Shortness of breath can manifest as orthopnea (inability to lie down flat due to breathlessness) and/or paroxysmal nocturnal dyspnea (episodes of severe sudden breathlessness at night). These are common presenting symptoms of chronic pulmonary edema due to left ventricular failure. The development of pulmonary edema may be associated with symptoms and signs of "fluid overload"; this is a non specific term to describe the manifestations of left ventricular failure on the rest of the body and includes peripheral edema (swelling of the legs, in general, of the "pitting" variety, wherein the skin is slow to return to normal when pressed upon), raised jugular venous pressure and hepatomegaly, where the liver is enlarged and may be tender or even pulsatile. Other signs include end-inspiratory crackles (sounds heard at the end of a deep breath) on auscultation and the presence of a third heart sound.

In the present invention it is demonstrated that Pro-Adrenomedullin or fragments thereof is associated with the severity of congestion and extent of edema, predicts decongestion and the applied dose of diuretics.

In one aspect of the present invention residual congestion is defined as a CCS ≥2 based on JVP, orthopnea and edema assessments by day 7.

In one aspect of the present invention decongestion is defined by any of the following three today's state-of-the-art markers, either alone or in combination:
- Diuretic response: defined as weight loss by day 4 per 40mg of diuretic dose.
- Hemoconcentration: coded as 0 (if there is a decline or no change in hemoglobin levels by day 4 compared to baseline) or 1 (if there is an increase in hemoglobin levels by day 4 compared to baseline).
- Significant residual congestion: defined as a CCS ≥2 based on JVP, orthopnea and edema assessments by day 7.

Subject matter of the present invention is a method for:
a) diagnosing congestion or assessing or monitoring the extent of congestion in a subject or,
b) predicting or determining or monitoring need of therapy or intervention of congestion or predicting or determining or monitoring the success of a therapy or intervention of congestion or guiding a therapy or intervention of congestion in a subject or,
c) predicting decongestion or residual congestion after therapy or intervention of congestion in a subject or,
d) assessing decongestion or residual congestion after therapy or intervention of congestion in a subject or,
e) assessing the decision on hospital discharge of a subject,
wherein said subject is a subject having acute heart failure and/or a subject having heart failure presenting with worsening signs and/or a subject with symptoms of heart failure or acute heart failure by using Pro-Adrenomedullin or fragments thereof of at least 5 amino acids as an early surrogate marker for congestion comprising:
- Determining the level of immunoreactive analyte by using at least one binder that binds to a region within the amino acid sequence of Pro-Adrenomedullin or fragments thereof of at least 5 amino acids in a bodily fluid obtained from said subject; and

a) Correlating said level of immunoreactive analyte with the extent of congestion in said subject or diagnosing congestion wherein an elevated level above a certain threshold is indicative of congestion or the extent of congestion or,
b) Correlating said level of immunoreactive analyte with the need or success of a therapy or intervention of congestion in said subject, wherein a level below a certain threshold is predictive for a success of therapy or intervention of congestion, and wherein a level above a certain threshold is indicative for need of therapy or intervention of congestion or,
c) Correlating said level of immunoreactive analyte with a prediction of decongestion or residual congestion after therapy or intervention of congestion wherein an elevated level above a certain threshold predicts residual congestion after therapy or intervention of congestion, whereas a level below a certain threshold predicts decongestion after therapy or,
d) Correlating said level of immunoreactive analyte with decongestion or residual congestion after therapy or intervention of congestion, wherein an elevated level above a certain threshold indicates residual congestion after therapy or intervention of congestion, whereas a level below a certain threshold indicates decongestion after therapy or intervention of congestion, or
e) Correlating said level of immunoreactive analyte with the assessment of the decision on hospital discharge, wherein an elevated level above a certain threshold means that the subject shall not be discharged and wherein a level below a certain threshold means that the subject may be discharged,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH₂ according to SEQ ID No.: 4 or ADM-Gly according to SEQ ID No.: 5 or CT-proADM according to SEQ ID No.: 6.

It can be seen from the examples that baseline levels of Pro-Adrenomedullin or fragment thereof, in particular ADM-NH₂ according to SEQ ID No.: 4, independently predict significant residual congestion e.g. by day 7, diuretic response by e.g. day 4, and hemoconcentration by e.g. day 4. There are very few baseline clinical variables or biomarkers associated with severity of congestion at baseline and Pro-Adrenomedullin or fragment thereof, in particular ADM-NH₂ according to SEQ ID No.: 4, appears to be, by far, the strongest. Pro-Adrenomedullin or fragment thereof, in particular ADM-NH₂ according to SEQ ID No.: 4, is strongly associated with severity of clinical congestion at baseline and independently predicts the presence of significant residual congestion by e.g. day 7. The data strongly support the notion that Pro-Adrenomedullin or fragment thereof, in particular ADM-NH₂ according to SEQ ID No.: 4, is a reliable, quantitative and early surrogate for congestion. This is an intriguing finding given that there is a huge unmet medical need for an accurate and reliable tool for the assessment of congestion in AHF patients, particularly for the ascertainment of presence of residual congestion pre-discharge. This, according to the present invention Pro-Adrenomedullin or fragment thereof, in particular ADM-NH₂ according to SEQ ID No.: 4, may be used for the ascertainment of presence of residual congestion pre-discharge.

Subject matter of the present invention is a method, wherein said extent of congestion is expressed as congestion score, in particular a clinical congestion score.

Subject matter of the present invention is a method, wherein said subject is stratified:
a) into groups of grades of congestion or,
b) into non-responder and/or responder, and/or bad responder to therapy or intervention of congestion or,
c) into a group with decongestion or into a group of residual congestion after therapy or intervention of congestion.

Subject matter of the present invention is a method, wherein said subject is stratified into groups of patients wherein one group comprises patients in need of therapy and the other group comprises patient that are not in need of therapy.

Previously, amongst others the markers BNP and proBNP have been considered as congestion marker. Surprisingly the biomarker Pro-Adrenomedullin or the respective fragments of the present invention are far superior than any other clinical or biochemical parameter used so far as congestion marker. This has been shown e.g. by Kremer et al. European Journal of Heart Failure (2017) 19 (Suppl S 1) 5-601, Kremer et al. studied the potential role of bio-ADM, a biomarker representing biologically active adrenomedullin, as a potential marker of congestion in ADHF, and evaluated added prognostic value on top of clinically assessed congestion. Bio-ADM was evaluated at baseline, days 2 and 7 in 1562 patients admitted for ADHF. Clinical congestion was assessed using a composite clinical congestion score (CCS) encompassing edema, orthopnea and jugular venous distension. This has been compared to other clinical and biochemical parameter s, including BNP. Amongst a large number of clinical and biochemical parameters, bio-ADM was the strongest predictor of clinically assessed congestion. Baseline bio-ADM levels, but not BNP levels, were strongly and independently associated with presence of significant residual congestion by day 7. In patients with residual congestion at day 7, bio-ADM levels remained high, while BNP levels decreased. Bio-ADM, but not BNP, was a strong independent predictor of 60-day heart failure rehospitalization, on top of clinically assessed congestion. Thus, the authors concluded that Bio-ADM is elevated in patients with ADHF and is a promising marker of (residual) congestion in patients admitted for ADHF. Bio-ADM was a strong and independent predictor of rehospitalization early after discharge.

In a specific embodiment of the invention said proADM and/or fragments thereof having at least 5 amino acids is/are selected from the group comprising:
SEQ ID No. 1 (proADM): 164 amino acids (22 - 185 of preproADM) 
ARLDVASEF RKKWNKWALS RGKRELRMSS SYPTGLADVK AGPAQTLIRP
QDMKGASRSP EDSSPDAARI RVKRYRQSMN NFQGLRSFGC RFGTCTVQKL
 
AHQIYQFTDK DKDNVAPRSK ISPQGYGRRR RRSLPEAGPG RTLVSSKPQA
HGAPAPPSGS APHFL
SEQ ID No. 2 (Proadrenomedullin N-20 terminal peptide, PAMP): amino acids 22 - 41 of preproADM
   ARLDVASEF RKKWNKWALS R
SEQ ID No. 3 (Midregional proAdrenomedullin, MR-proADM): amino acids 45 - 92 of preproADM
   ELRMSS SYPTGLADVK AGPAQTLIRP QDMKGASRSP EDSSPDAARI RV
SEQ ID No. 4 (mature Adrenomedullin (mature ADM); amidated ADM; bio-ADM; hADM): amino acids 95 - 146 -CONH₂ 
YRQSMN NFQGLRSFGC RFGTCTVQKL AHQIYQFTDK DKDNVAPRSK ISPQGY -
CONH2
SEQ ID No. 5 (Adrenomedullin 1-52-Gly (ADM 1-52-Gly)): amino acids 95 - 147 of preproADM
   YRQSMN NFQGLRSFGC RFGTCTVQKL AHQIYQFTDK DKDNVAPRSK ISPQGYG
SEQ ID No. 6 (C-terminal proAdrenomedullin, CT-proADM): amino acids 148 - 185 of preproADM
   RRR RRSLPEAGPG RTLVSSKPQA HGAPAPPSGS APHFL

In a specific embodiment of the invention said proADM and/or fragments thereof having at least 5 amino acids is/are selected from the group comprising mature ADM-NH₂ (SEQ ID No. 4), ADM 1-52-Gly (SEQ ID No. 5), MR-proADM (SEQ ID No. 3) and CT-proADM (SEQ ID No. 6).

In a specific embodiment of the invention either the level of mature ADM-NH₂ (SEQ ID No. 4) and/or ADM 1-52-Gly (SEQ ID No. 5) - immunoreactivity or the level of MR-proADM (SEQ ID No. 3) immunoreactivity or the level of CT-proADM (SEQ ID No. 6) immunoreactivity is determined and correlated with the need of said patient for therapy or intervention, wherein said patient is identified as having such a need if the level of mature ADM-NH₂ (SEQ ID No. 4) and/or ADM 1-52-Gly (SEQ ID No. 5) - immunoreactivity or the level of MR-proADM (SEQ ID No. 3) immunoreactivity or the level of CT-proADM (SEQ ID No. 6) immunoreactivity in the bodily fluid of said subject is above a threshold.

In a specific embodiment of the invention the level of proADM and/or fragments thereof is determined by using at least one binder selected from the group: a binder that binds to a region comprised within the following sequence of mature ADM-NH₂ (SEQ ID No. 4) and/or ADM 1-52-Gly (SEQ ID No. 5) and a second binder that binds to a region comprised within the sequence of mature ADM-NH₂ (SEQ ID NO. 4) and/or ADM 1-52-Gly (SEQ ID No. 5).

In a specific embodiment of the invention the level of proADM and/or fragments thereof is determined by using at least one binder selected from the group: a binder that binds to a region comprised within the sequence of MR-proADM (SEQ ID No. 3) and a second binder that binds to a region comprised within the sequence of MR-proADM (SEQ ID No. 3).

In a specific embodiment of the invention the level of pro-ADM and/or fragments thereof is determined by using at least one binder selected from the group: a binder that binds to a region comprised within the sequence of CT-proADM (SEQ ID No. 6) and a second binder that binds to a region comprised within the sequence of CT-pro-ADM (SEQ ID No. 6).

Subject matter in a particular embodiment of the present invention is a method, according to the present invention wherein said fragment may be selected from MR-proADM according to SEQ ID No.: 3 or mature ADM-NH₂ according to SEQ ID No.: 4.

Subject matter of the present invention is a method according to the present invention, wherein the level of Pro-Adrenomedullin or fragments thereof of at least 5 amino acids is determined by using a binder to Pro-Adrenomedullin or fragments thereof of at least 5 amino acids.

Subject matter of the present invention is method, according to the present invention wherein the binder is selected from the group comprising an antibody, an antibody fragment or a non-Ig-Scaffold binding to Pro-Adrenomedullin or fragments thereof of at least 5 amino acids.

A bodily fluid according to the present invention is in one particular embodiment a blood sample. A blood sample may be selected from the group comprising whole blood, serum and plasma. In a specific embodiment of the invention said sample is selected from the group comprising human citrate plasma, heparin plasma and EDTA plasma.

Subject matter of the present invention is a method, according to the present invention wherein said determination of Pro-Adrenomedullin or fragments thereof of at least 5 amino acids is performed more than once in one patient.

Subject matter of the present invention is a method, wherein the sample is taken on admission to a hospital or prior discharge of the hospital.

Subject matter of the present invention is a method, according to the present invention wherein said monitoring is performed in order to evaluate the response of said subject to preventive and/or therapeutic measures taken.

Subject matter of the present invention is a method, according to the present invention wherein said method is used in order to stratify said subjects into congestion grade groups.

Subject matter of the present invention is a method according to the present invention wherein said level of Pro-Adrenomedullin or fragments thereof are correlated with a risk of death or an adverse event in subject having acute heart failure or heart failure, wherein an elevated level above a certain threshold is predictive for an enhanced risk of death or adverse events.

In a specific embodiment of the invention, an assay is used for determining the level of proADM and/or fragments thereof having at least 5 amino acids, wherein the assay sensitivity of said assay is able to quantify the mature ADM-NH₂ of healthy subjects and is < 70 pg/ml, preferably < 40 pg/ml and more preferably < 10 pg/ml.

In a specific embodiment of the invention, an assay is used for determining the level of proADM and/or fragments thereof having at least 5 amino acids, wherein the assay sensitivity of said assay is able to quantify MR-proADM of healthy subjects and is < 0.5 nmol/L, preferably < 0.4 nmol/L and more preferably < 0.2 nmol/L.

In a specific embodiment of the invention, an assay is used for determining the level of proADM and/or fragments thereof having at least 5 amino acids, wherein the assay sensitivity of said assay is able to quantify CT-proADM of healthy subjects and is < 100 pmol/L, preferably < 75 pmol/L and more preferably < 50 pmol/L.

In a specific embodiment of the invention, said binder exhibits a binding affinity to proADM and/or fragments thereof of at least 10⁷ M⁻¹, preferred 10⁸ M⁻¹, preferred affinity is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention.

To determine the affinity of the antibodies to Adrenomedullin, the kinetics of binding of Adrenomedullin to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare), (Lorenz et al. 2011. Antimicrob Agents Chemother. 55 (1): 165-173*).*

In a specific embodiment of the invention, said binder is selected from the group comprising an antibody or an antibody fragment or a non-Ig scaffold binding to proADM and/or fragments thereof.

In a specific embodiment of the invention, an assay is used for determining the level of proADM and/or fragments thereof having at least 5 amino acids, wherein such assay is a sandwich assay, preferably a fully automated assay.

In one embodiment of the invention it may be a so-called POC-test (point-of-care) that is a test technology, which allows performing the test within less than 1 hour near the patient without the requirement of a fully automated assay system. One example for this technology is the immunochromatographic test technology.

In one embodiment of the invention such an assay is a sandwich immunoassay using any kind of detection technology including but not restricted to enzyme label, chemiluminescence label, electrochemiluminescence label, preferably a fully automated assay. In one embodiment of the invention such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, BiomerieuxVidas^{®}, Alere Triage^{®}.

A variety of immunoassays are known and may be used for the assays and methods of the present invention, these include: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immuno-chromotography assays.

In a specific embodiment of the invention, at least one of said two binders is labeled in order to be detected.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

In a preferred embodiment, said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person *(*The Immunoassay Handbook, Ed. David Wild, Elsevier LTD, Oxford: 3rd ed. (May 2005*);* Hultschig et al. 2006. Curr Opin Chem Biol. 10 (1):4-10).

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in *(*Kirk-Othmer, Encyclopedia of chemical technology, 4th ed. 1993. John Wiley & Sons, Vol.15: 518-562*, incorporated herein by reference, including citations on pages 551-562).* Preferred chemiluminescent dyes are acridinium esters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present invention, "binder molecules" are molecules which may be used to bind target molecules or molecules of interest, *i.e.* analytes *(i.e.* in the context of the present invention ADM-NH₂ and/ or proADM and fragments thereof), from a sample. Binder molecules have thus to be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, binder molecules may for instance be selected from the group comprising a nucleic acid molecule, a carbohydrate molecule, a PNA molecule, a protein, an antibody, a peptide or a glycoprotein. Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

Chemiluminescent label may be acridinium ester label, steroid labels involving isoluminol labels and the like.

Enzyme labels may be lactate dehydrogenase (LDH), creatine kinase (CPK), alkaline phosphatase, aspartate aminotransferase (AST), alanine aminotransferase (ALT), acid phosphatase, glucose-6-phosphate dehydrogenase and so on.

In one embodiment of the invention at least one of said two binders is bound to a solid phase as magnetic particles, and polystyrene surfaces.

In a specific embodiment of the invention at least one of said two binders is bound to a solid phase.

In a specific embodiment of the invention the threshold is within a threshold range for plasma ADM-NH₂ that is between 50 and 100 pg/ml, preferably between 60 and 90 pg/ml, most preferred a threshold of 70 pg/ml is applied.

In a specific embodiment of the invention the threshold is within a threshold range for plasma MR-proADM that is between 0.5 and 1.5 nmol/L, preferably between 0.7 and 1 nmol/L, most preferred a threshold of 0.8 nmol/L is applied.

In a specific embodiment of the invention the threshold is within a threshold range for plasma CT-proADM that is between 85 and 350 pmol/L, preferably between 100 and 250 pmol/L, most preferred a threshold of 150 pmol/L is applied.

The ADM-NH₂ levels of the present invention or proADM levels or fragments thereof, respectively, have been determined with the described ADM-NH₂ assay, as outlined in the examples (or proADM or fragments thereof assays, respectively). The mentioned threshold values above might be different in other assays, if these have been calibrated differently from the assay systems used in the present invention. Therefore, the mentioned cut-off values above shall apply for such differently calibrated assays accordingly, taking into account the differences in calibration. One possibility of quantifying the difference in calibration is a method comparison analysis (correlation) of the assay in question with the respective biomarker assay used in the present invention by measuring the respective biomarker (e.g. bio-ADM) in samples using both methods. Another possibility is to determine with the assay in question, given this test has sufficient analytical sensitivity, the median biomarker level of a representative normal population, compare results with the median biomarker levels as described in the literature and recalculate the calibration based on the difference obtained by this comparison. With the calibration used in the present invention, samples from normal (healthy) subjects have been measured: median plasma bio-ADM (mature ADM-NH₂) was 24.7 pg/ml, the lowest value 11 pg/ml and the 99^{th} percentile 43 pg/ml. Alternatively, commercially available control samples could be used for adjustment of different calibrations (e.g. ICI Diagnostics, Berlin, Germany).

The plasma median MR-proADM concentration in normal (healthy) subjects was 0.41 (interquartile range 0.23 - 0.64) nmol/L *(*Smith et al. 2009. Clin Chem 55:1593-1595) using the automated sandwich fluorescence assay for the detection of MR-proADM as described in Caruhel et al. (Caruhel et al. 2009. Clin Biochem 42:725-8)*.*

The plasma median concentration of CT-proADM in normal healthy subjects (n=200) was 77.6 pmol/L (min 46.6 pmol/L, max 136.2 pmol/L) and the 95% percentile was 113.8 pmol/L (EP 2 111 552 B1).

In a specific embodiment of the invention a threshold for plasma ADM-NH₂ is the 5fold median concentration, preferably the 4fold median concentration, more preferred the 3fold median concentration, most preferred the 2fold median concentration of a normal healthy population.

In a specific embodiment of the invention a threshold for plasma MR-proADM is the 5fold median concentration, preferably the 4fold median concentration, more preferred the 3fold median concentration, most preferred the 2fold median concentration of a normal healthy population.

In a specific embodiment of the invention a threshold for plasma CT-proADM is the 5fold median concentration, preferably the 4fold median concentration, more preferred the 3fold median concentration, most preferred the 2fold median concentration of a normal healthy population.

An antibody according to the present invention is a protein including one or more polypeptides substantially encoded by immunoglobulin genes that specifically binds an antigen. The recognized immunoglobulin genes include the kappa, lambda, alpha (IgA), gamma (IgGi, IgG₂, IgG₃, IgG₄), delta (IgD), epsilon (IgE) and mu (IgM) constant region genes, as well as the myriad immunoglobulin variable region genes. Full-length immunoglobulin light chains are generally about 25 Kd or 214 amino acids in length. Full-length immunoglobulin heavy chains are generally about 50 Kd or 446 amino acid in length. Light chains are encoded by a variable region gene at the NH2-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')₂, as well as bifunctional hybrid antibodies and single chains *(e.g.,* Lanzavecchia et al. 1987. Eur. J. Immunol. 17:105*;* Huston et al. 1988, Proc. Natl. Acad. Sci. U.S.A., 85:5879-5883*;* Bird et al. 1988, Science 242:423-426*;* Hood et al., Immunology, Benjamin, N.Y., 2nd ed., 1984*;* Hunkapiller and Hood 1986. Nature 323:15-16). An immunoglobulin light or heavy chain variable region includes a framework region interrupted by three hypervariable regions, also called complementarity determining regions (CDR's) (see, Sequences of Proteins of Immunological Interest, E. Kabatet al., U.S. Department of Health and Human Services, 1983*).* As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art *(e.g.,* see U.S. Patent No. 5,807,715). A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, *i.e.,* at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions, which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering *(e.g.,* see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.,* Dower et al., PCT Publication No. WO91/17271*;* McCafferty et al., PCT Publication No. WO92/001047*; and* Winter, PCT Publication No. WO92/20791), or selected from a human combinatorial monoclonal antibody library (see the Morphosys website). Human antibodies can also be prepared by using transgenic animals carrying a human immunoglobulin gene (for example, see Lonberget al., PCT Publication No. WO93/12227*; and* Kucherlapati, PCT Publication No. WO91/10741)*.*

Thus, the antibody may have the formats known in the art. Examples are human antibodies, monoclonal antibodies, humanized antibodies, chimeric antibodies, CDR-grafted antibodies. In a preferred embodiment antibodies according to the present invention are recombinantly produced antibodies as *e.g.* IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLXdomains, *e.g.* Fab-dHLX-FSx2; F(ab')2-fragments, scFv-fragments, multimerized multivalent or/and multispecificscFv-fragments, bivalent and/or bispecificdiabodies, BITE^{®} (bispecific T-cell engager), trifunctional antibodies, polyvalent antibodies, *e.g.* from a different class than G; single-domain antibodies, *e.g.* nanobodies derived from camelid or fish immunoglobulines and numerous others.

In addition to antibodies other biopolymer scaffolds are well known in the art to complex a target molecule and have been used for the generation of highly target specific biopolymers. Examples are aptamers, spiegelmers, anticalins and conotoxins.

In a preferred embodiment the antibody format is selected from the group comprising Fv fragment, scFv fragment, Fab fragment, scFab fragment, (Fab)2 fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is the scFab format.

Non-Ig scaffolds may be protein scaffolds and may be used as antibody mimics as they are capable to bind to ligands or antigenes. Non-Ig scaffolds may be selected from the group comprising tetranectin-based non-Ig scaffolds *(e.g.* described in US 2010/0028995*),* fibronectin scaffolds *(e.g.* described in EP 1 266 025*),* lipocalin-based scaffolds *(e.g.* described in WO 2011/154420*);* ubiquitin scaffolds *(e.g.* described in WO 2011/073214*),* transferring scaffolds *(e.g.* described in US 2004/0023334*),* protein A scaffolds *(e.g.* described in EP 2 231 860), ankyrin repeat based scaffolds *(e.g.* described in WO 2010/060748*),* microprotein scaffolds (preferably microproteins forming a cystine knot) *(e.g.* described in EP 2 314 308*),* Fyn SH3 domain based scaffolds *(e.g.* described in WO 2011/023685*)* EGFR-A-domain based scaffolds *(e.g.* described in WO 2005/040229*)* and Kunitz domain based scaffolds *(e.g.* described in EP 1941867*).*

In one embodiment of the invention antibodies according to the present invention may be produced as follows:
A Balb/c mouse was immunized with ADM-100µg Peptide-BSA-Conjugate at day 0 and 14 (emulsified in 100µl complete Freund's adjuvant) and 50µg at day 21 and 28 (in 100µl incomplete Freund's adjuvant). Three days before the fusion experiment was performed, the animal received 50µg of the conjugate dissolved in 100µl saline, given as one intraperitoneal and one intravenous injection.

Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium [RPMI 1640 culture medium supplemented with 20% fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined (see also Lane 1985. J. Immunol. Meth. 81: 223-228*;* Ziegler, B. et al. 1996 Horm. Metab. Res. 28: 11-15).

Antibodies may be produced by means of phage display according to the following procedure:
The human naive antibody gene libraries HAL7/8 were used for the isolation of recombinant single chain F-Variable domains (scFv) against adrenomedullin peptide. The antibody gene libraries were screened with a panning strategy comprising the use of peptides containing a biotin tag linked via two different spacers to the adrenomedullin peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing (see Hust et al. 2011. Journal of Biotechnology 152: 159-170*;* Schütte et al. 2009. PLoS One 4, e6625).

Humanization of murine antibodies may be conducted according to the following procedure:
For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modelling (Almagro et al. 2008. Front Biosci. 2008; 13:1619-33*).*

Subject matter of the invention is also a method, according the present invention wherein said level of Pro- Adrenomedullin or fragments thereof is used for guidance of therapy or intervention, wherein therapy or intervention is indicated if said level of Pro- Adrenomedullin or fragments is above a certain threshold and wherein therapy or intervention is not indicated if said level of Pro- Adrenomedullin or fragments is below a certain threshold. Threshold and threshold ranges are given above.

The following embodiments are subject of the present invention:
1. A method for
   a) diagnosing congestion or assessing or monitoring the extent of congestion in a subject or,
   b) predicting or determining or monitoring need of therapy or intervention of congestion or predicting or determining or monitoring the success of a therapy or intervention of congestion or guiding a therapy or intervention of congestion in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is a subject having acute heart failure that is either new-onset AHF or acute decompensated HF or acute decompensated chronic HF or wherein said subject is a subject having chronic heart failure with worsening signs/symptoms of chronic heart failure and wherein by using Pro-Adrenomedullin or fragments thereof of at least 5 amino acids is used as an early surrogate marker for congestion comprising:
   - Determining the level of Pro-Adrenomedullin or fragments thereof of at least 5 amino acids in a bodily fluid obtained from said subject; and

   a) Correlating said level of Pro-Adrenomedullin or fragments thereof with the extent of congestion in said subject or diagnosing congestion wherein an elevated level above a certain threshold is indicative of congestion, or the extent of congestion or,
   b) Correlating said level of Pro-Adrenomedullin or fragments thereof with the success of a therapy or intervention of congestion in said subject, wherein a level below a certain threshold is predictive for a success of therapy or intervention of congestion, and wherein a level above a certain threshold is indicative for need of therapy or intervention of congestion, or
   c) Correlating said level of Pro-Adrenomedullin or fragments thereof with the prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
   d) Correlating said level of Pro-Adrenomedullin or fragments thereof with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
   e) Correlating said level of Pro-Adrenomedullin or fragments thereof with the assessment of the decision on hospital discharge, wherein an elevated level above a certain threshold means that the subject shall not be discharged and wherein a level below a certain threshold means that the subject may be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6.
2. A method according to item 1 for
   a) diagnosing congestion or assessing or monitoring the extent of congestion in a subject or,
   b) predicting or determining or monitoring need of therapy or intervention of congestion or predicting or determining or monitoring the success of a therapy or intervention of congestion or guiding a therapy or intervention of congestion in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is subject having acute heart failure that is either new-onset AHF or acute decompensated HF or acute decompensated chronic HF or wherein said subject is a subject having chronic heart failure with worsening signs/symptoms of chronic heart failure and wherein Pro-Adrenomedullin or fragments thereof of at least 5 amino acids is used as an early surrogate marker for congestion comprising:
   - Determining the level of immunoreactive analyte by using at least one binder that binds to a region within the amino acid sequence of Pro-Adrenomedullin or fragments thereof of at least 5 amino acids in a bodily fluid obtained from said subject; and

   a) Correlating said level of immunoreactive analyte with the extent of congestion in said subject or diagnosing congestion wherein an elevated level above a certain threshold is indicative of congestion or the extent of congestion or,
   b) Correlating said level of immunoreactive analyte with success of a therapy or intervention of congestion in said subject, wherein a level below a certain threshold is predictive for a success of therapy or intervention, and wherein a level above a certain threshold is indicative for need of therapy or intervention of congestion or
   c) Correlating said level of immunoreactive analyte with a prediction of decongestion or residual congestion after therapy or intervention of congestion wherein an elevated level above a certain threshold predicts residual congestion after therapy or intervention of congestion whereas a level below a certain threshold predicts decongestion after therapy or
   d) Correlating said level of immunoreactive analyte with decongestion or residual congestion after therapy or intervention of congestion wherein an elevated level above a certain threshold indicates residual congestion after therapy or intervention of congestion whereas a level below a certain threshold indicates decongestion after therapy or intervention of congestion
   e) Correlating said level of immunoreactive analyte with the assessment of the decision on hospital discharge wherein an elevated level above a certain threshold means that the subject shall not be discharged and wherein a level below a certain threshold means that the subject shall not be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH₂ according to SEQ ID No.: 4 or ADM-Gly according to SEQ ID No.: 5 or CT-proADM according to SEQ ID No.: 6.
3. A method according to items 1 or 2, wherein said extent of congestion is expressed as congestion score, in particular a clinical congestion score.
4. A method of any of items 1 to 3, wherein said subject is stratified
   a) into groups of grades of congestion or,
   b) into non-responder and/or responder, and/or bad responder to therapy or intervention of congestion or,
   c) into a group with decongestion or into a group of residual congestion after therapy or intervention of congestion.
5. A method according to any of items 1 to 4 wherein said fragment may be selected from MR-proADM according to SEQ ID No.: 3 or ADM-NH₂ according to SEQ ID No.: 4.
6. A method according to any of items 1 to 5, wherein the level of Pro-Adrenomedullin or fragments thereof of at least 5 amino acids is determined by using a binder to Pro-Adrenomedullin or fragments thereof of at least 5 amino acids.
7. A method according to item 6, wherein the binder is selected from the group comprising an antibody, an antibody fragment or a non-Ig-Scaffold binding to Pro-Adrenomedullin or fragments thereof of at least 5 amino acids.
8. A method according to any of the preceding items, wherein said threshold is within a threshold range that is a threshold range for plasma ADM-NH₂ according to SEQ ID No.: 4 between 50 and 100 pg/ml and for plasma MR-proADM between 0.5 and 1.5 nmol/L, and for plasma CT-proADM between 85 and 350 pmol/L.
9. A method according to any of items 1 to 8, wherein said determination of Pro-Adrenomedullin or fragments thereof of at least 5 amino acids is performed more than once in one patient.
10. A method according to any of items 1 to 9, wherein the sample is taken on admission to a hospital or prior to discharge of the hospital.
11. A method according to any of items 1 to 10, wherein said monitoring is performed in order to evaluate the response of said subject to preventive and/or therapeutic measures taken.
12. A method according to any of items 1 to 11 in order to stratify said subjects into congestion grade groups.
13. A method according to any of items 1 to 12, wherein said level of Pro- Adrenomedullin or fragments thereof or said level of immunoreactive analyte is correlated with a risk of death or an adverse event in subject having acute heart failure that is either new-onset AHF or acute decompensated HF or acute decompensated chronic HF or wherein said subject is a subject having chronic heart failure with worsening signs/symptoms of chronic heart failure, wherein an elevated level above a certain threshold is predictive for an enhanced risk of death or adverse events.
14. A method according to any of items 1 to 13, wherein said level of Pro- Adrenomedullin or fragments thereof or said level of immunoreactive analyte is used for guidance of therapy or intervention, wherein therapy or intervention is indicated if said level of Pro- Adrenomedullin or fragments is above a certain threshold and wherein, therapy or intervention is not indicated if said level of Pro- Adrenomedullin or fragments is below a certain threshold.
15. A method according to any of items 1 to 14 wherein said level of Pro- Adrenomedullin or fragments thereof or said level of immunoreactive analyte is used for determining the need of therapy or intervention.
16. A method according to any of items 1 to 15 wherein said intervention or therapy is selected from the group comprising administration of diuretics, administration of inotropes, administration of vasodilators, ultrafiltration.
17. Use of a binder in the manufacture of a reagent or a medicament or a kit for
   a) diagnosing congestion or assessing or monitoring the extent of congestion in a subject or,
   b) predicting or determining or monitoring need of therapy or intervention or predicting or determining or monitoring the success of a therapy or intervention of congestion or guiding a therapy or intervention in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is a subject having acute heart failure that is either new-onset AHF or acute decompensated HF or acute decompensated chronic HF or wherein said subject is a subject having chronic heart failure with worsening signs/symptoms of chronic heart failure, and wherein the binder binds to Pro-Adrenomedullin or fragments thereof of at least 5 amino acids and can determine the level of Pro-Adrenomedullin or fragments thereof of at least 5 amino acids in a bodily fluid sample obtained from said subject, and the Pro-Adrenomedullin or fragments thereof of at least 5 amino acids are used as an early surrogate marker for congestion; and wherein
   a) said level of Pro-Adrenomedullin or fragments thereof is correlated with the extent of congestion in said subject or diagnosing congestion, wherein an elevated level above a certain threshold is indicative of congestion or,
   b) said level of Pro-Adrenomedullin or fragments thereof is correlated with success of a therapy or intervention in said subject, wherein a level below a certain threshold is predictive for a success of therapy or intervention, and wherein a level above a certain threshold is indicative for need of therapy or intervention or,
   c) said level of Pro-Adrenomedullin or fragments thereof is correlated with a prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
   d) said level of Pro-Adrenomedullin or fragments thereof is correlated with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
   e) said level of Pro-Adrenomedullin or fragments thereof is correlated with the assessment of the decision on hospital discharge, wherein an elevated level above a certain threshold means that the subject shall not be discharged and wherein a level below a certain threshold means that the subject may be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH2 according to SEQ ID No.: 4 or ADM-Gly according to SEQ ID No.: 5 or CT-proADM according to SEQ ID No.: 6.
18. Use of at least one binder in the manufacture of a reagent or a medicament or a kit for
   a) diagnosing congestion or assessing or monitoring the extent of congestion in a subject or,
   b) predicting or determining or monitoring need of therapy or intervention or predicting or determining or monitoring the success of a therapy or intervention of congestion or guiding a therapy or intervention in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is a subject having acute heart failure that is either new-onset AHF or acute decompensated HF or acute decompensated chronic HF or wherein said subject is a subject having chronic heart failure with worsening signs/symptoms of chronic heart failure, wherein the binder binds to a region within the amino acid sequence of Pro-Adrenomedullin or fragments thereof of at least 5 amino acids and can determine the level of immunoreactive analyte in a bodily fluid sample obtained from said subject, and the Pro-Adrenomedullin or fragments thereof of at least 5 amino acids are used as an early surrogate marker for congestion; and wherein
   a) said level of immunoreactive analyte is correlated with the extent of congestion in said subject or diagnosing congestion, wherein an elevated level above a certain threshold is indicative of congestion or,
   b) said level of immunoreactive analyte is correlated with success of a therapy or intervention in said subject, wherein a level below a certain threshold is predictive for a success of therapy or intervention, and wherein a level above a certain threshold is indicative for need of therapy or intervention or,
   c) said level of immunoreactive analyte is correlated with a prediction of decongestion or residual congestion after therapy or intervention of congestion, wherein an elevated level above a certain threshold predicts residual congestion after therapy or intervention of congestion whereas a level below a certain threshold predicts decongestion after therapy or,
   d) said level of immunoreactive analyte is correlated with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
   e) said level of immunoreactive analyte is correlated with the assessment of the decision on hospital discharge wherein an elevated level above a certain threshold means that the subject shall not be discharged and wherein a level below a certain threshold means that the subject may be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH2 according to SEQ ID No.: 4 or ADM-Gly according to SEQ ID No.: 5 or CT-proADM according to SEQ ID No.: 6.
19. The use according to item 17 or 18, wherein said extent of congestion is expressed as congestion score, in particular a clinical congestion score.
20. The use according to any of items 17 to 19, wherein said subject is stratified
   a) into groups of grades of congestion or,
   b) into non-responder and/or responder, and/or bad responder to therapy or intervention of congestion or,
   c) into a group with decongestion or into a group of residual congestion after therapy or intervention of congestion.
21. The use according to any of items 17 to 20 wherein said fragment may be selected from MR-proADM according to SEQ ID No.: 3 or ADM-NH2 according to SEQ ID No.: 4.
22. The use according to any of items 17 to 21, wherein the binder is selected from the group comprising an antibody, an antibody fragment or a non-Ig-Scaffold binding to Pro-Adrenomedullin or fragments thereof of at least 5 amino acids.
23. The use according to any of the preceding items, wherein said threshold is within a threshold range that is a threshold range for plasma ADM-NH2 according to SEQ ID No.: 4 between 50 and 100 pg/ml and for plasma MR-proADM between 0.5 and 1.5 nmol/L, and for plasma CT-proADM between 85 and 350 pmol/L.
24. The use according to any of items 17 to 23, wherein said determination of Pro-Adrenomedullin or fragments thereof of at least 5 amino acids is performed more than once in one subject.
25. The use according to any of items 17 to 24, wherein the sample is taken at admission to a hospital or discharge of hospital.
26. The use according to any of items 17 to 25, wherein said monitoring is performed in order to evaluate the response of said subject to preventive and/or therapeutic measures taken.
27. The use according to any of items 17 to 26 in order to stratify said subjects into congestion grade groups.
28. The use according to any of items 17 to 27 wherein said level of Pro-Adrenomedullin or fragments thereof or said level of immunoreactive analyte is correlated with a risk of death or an adverse event in subject having acute heart failure, wherein an elevated level above a certain threshold is predictive for an enhanced risk of death or adverse events.
29. The use according to any of items 17 to 28 wherein said level of Pro-Adrenomedullin or fragments thereof or said level of immunoreactive analyte is used for guidance of therapy or intervention, wherein therapy or intervention is indicated if said level of Pro-Adrenomedullin or fragments is above a certain threshold and wherein, therapy or intervention, is not indicated if said level of Pro-Adrenomedullin or fragments is below a certain threshold.
30. The use according to any of items 17 to 29 wherein said level of Pro-Adrenomedullin or fragments thereof or said level of immunoreactive analyte is used for determining the need of therapy or intervention.
31. The use according to any of items 17 to 30 wherein said intervention or therapy is selected from the group comprising administration of diuretics, administration of inotropes, administration of vasodilators, ultrafiltration.
32. A kit or reagent or medicament for
   a) diagnosing congestion or assessing or monitoring the extent of congestion in a subject or,
   b) predicting or determining or monitoring need of therapy or intervention or predicting or determining or monitoring the success of a therapy or intervention of congestion or guiding a therapy or intervention in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is a subject having acute heart failure that is either new-onset AHF or acute decompensated HF or acute decompensated chronic HF or wherein said subject is a subject having chronic heart failure with worsening signs/symptoms of chronic heart failure, and wherein the kit or reagent or medicament comprises a binder binding to Pro-Adrenomedullin or fragments thereof of at least 5 amino acids, and the binder can determine the level of Pro-Adrenomedullin or fragments thereof of at least 5 amino acids in a bodily fluid sample obtained from said subject; and wherein
   a) said level of Pro-Adrenomedullin or fragments thereof is correlated with the extent of congestion in said subject or diagnosing congestion, wherein an elevated level above a certain threshold is indicative of congestion or,
   b) said level of Pro-Adrenomedullin or fragments thereof is correlated with success of a therapy or intervention in said subject, wherein a level below a certain threshold is predictive for a success of therapy or intervention, and wherein a level above a certain threshold is indicative for need of therapy or intervention or,
   c) said level of Pro-Adrenomedullin or fragments thereof is correlated with a prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
   d) said level of Pro-Adrenomedullin or fragments thereof is correlated with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
   e) said level of Pro-Adrenomedullin or fragments thereof is correlated with the assessment of the decision on hospital discharge, wherein an elevated level above a certain threshold means that the subject shall not be discharged and wherein a level below a certain threshold means that the subject may be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH2 according to SEQ ID No.: 4 or ADM-Gly according to SEQ ID No.: 5 or CT-proADM according to SEQ ID No.: 6.
33. A kit or reagent or medicament for
   a) diagnosing congestion or assessing or monitoring the extent of congestion in a subject or,
   b) predicting or determining or monitoring need of therapy or intervention or predicting or determining or monitoring the success of a therapy or intervention of congestion or guiding a therapy or intervention in a subject or,
   c) predicting decongestion or residual congestion after therapy or intervention of congestion in a subject or,
   d) assessing decongestion or residual congestion after therapy or intervention of congestion in a subject or,
   e) assessing the decision on hospital discharge of a subject,
   wherein said subject is a subject having acute heart failure that is either new-onset AHF or acute decompensated HF or acute decompensated chronic HF or wherein said subject is a subject having chronic heart failure with worsening signs/symptoms of chronic heart failure, and wherein the kit or reagent or medicament comprises at least one binder that binds to a region within the amino acid sequence of Pro-Adrenomedullin or fragments thereof of at least 5 amino acids, and the binder can determine the level of immunoreactive analyte in a bodily fluid sample obtained from said subject; and wherein
   a) said level of immunoreactive analyte is correlated with the extent of congestion in said subject or diagnosing congestion, wherein an elevated level above a certain threshold is indicative of congestion or,
   b) said level of immunoreactive analyte is correlated with success of a therapy or intervention in said subject, wherein a level below a certain threshold is predictive for a success of therapy or intervention, and wherein a level above a certain threshold is indicative for need of therapy or intervention or,
   c) said level of immunoreactive analyte is correlated with a prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
   d) said level of immunoreactive analyte is correlated with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
   e) said level of immunoreactive analyte is correlated with the assessment of the decision on hospital discharge, wherein an elevated level above a certain threshold means that the subject shall not be discharged and wherein a level below a certain threshold means that the subject may be discharged,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or ADM-NH2 according to SEQ ID No.: 4 or ADM-Gly according to SEQ ID No.: 5 or CT-proADM according to SEQ ID No.: 6.
34. The kit or reagent or medicament according to item 32 or 33, wherein said fragment may be selected from MR-proADM according to SEQ ID No.: 3 or ADM-NH2 according to SEQ ID No.: 4.
35. The kit or reagent or medicament according to any one of items 32 - 34, wherein the binder is selected from the group comprising an antibody, an antibody fragment or a non-Ig-Scaffold binding to Pro-Adrenomedullin or fragments thereof of at least 5 amino acids
36. The kit or reagent or medicament according to any one of items 32 - 35, wherein said threshold is within a threshold range that is a threshold range for plasma ADM-NH2 according to SEQ ID No.: 4 between 50 and 100 pg/ml and for plasma MR-proADM between 0.5 and 1.5 nmol/L, and for plasma CT-proADM between 85 and 350 pmol/L.
   A1. A method for
      a) diagnosing congestion or assessing or monitoring the extent of congestion in a subject or,
      b) predicting or determining or monitoring need of therapy or intervention of congestion or predicting or determining or monitoring the success of a therapy or intervention of congestion or guiding a therapy or intervention of congestion in a subject or,
      c) predicting decongestion or residual congestion after therapy or intervention of congestion in a subject or,
      d) assessing decongestion or residual congestion after therapy or intervention of congestion in a subject or,
      e) assessing the decision on hospital discharge of a subject,

      wherein said subject is subject having acute heart failure that is either new-onset AHF or acute decompensated HF or acute decompensated chronic HF or wherein said subject is a subject having chronic heart failure with worsening signs/symptoms of chronic heart failure and
      wherein Pro-Adrenomedullin or fragments thereof of at least 5 amino acids is used as a marker for congestion and
      wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH2 according to SEQ ID No.: 4 or ADM-Gly according to SEQ ID No.: 5 or CT-proADM according to SEQ ID No.: 6.
   A2. A method of item 1 comprising the steps:
      - Determining the level of Pro-Adrenomedullin or fragments thereof of at least 5 amino acids in a bodily fluid obtained from said subject; and

      a) Correlating said level of Pro-Adrenomedullin or fragments thereof with the extent of congestion in said subject or diagnosing congestion, wherein an elevated level above a certain threshold is indicative of congestion or the extent of congestion or,
      b) Correlating said level of Pro-Adrenomedullin or fragments thereof with success of a therapy or intervention of congestion in said subject, wherein a level below a certain threshold is predictive for a success of therapy or intervention of congestion, and wherein a level above a certain threshold is indicative for need of therapy or intervention of congestion or,
      c) Correlating said level of Pro-Adrenomedullin or fragments thereof with a prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
      d) Correlating said level of Pro-Adrenomedullin or fragments thereof with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
      e) Correlating said level of Pro-Adrenomedullin or fragments thereof with the assessment of the decision on hospital discharge, wherein an elevated level above a certain threshold means that the subject shall not be discharged and wherein a level below a certain threshold means that the subject may be discharged,
      wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6.
   A3. A method according to item 1 comprising the steps:
      - Determining the level of immunoreactive analyte by using at least one binder that binds to a region within the amino acid sequence of Pro-Adrenomedullin or fragments thereof of at least 5 amino acids in a bodily fluid obtained from said subject; and

      a) Correlating said level of immunoreactive analyte with the extent of congestion in said subject or diagnosing congestion, wherein an elevated level above a certain threshold is indicative of congestion or the extent of congestion, or
      b) Correlating said level of immunoreactive analyte with success of a therapy or intervention of congestion in said subject, wherein a level below a certain threshold is predictive for a success of therapy or intervention of congestion, and wherein a level above a certain threshold is indicative for need of therapy or intervention of congestion, or
      c) Correlating said level of immunoreactive analyte with a prediction of decongestion or residual congestion after therapy or intervention of congestion, wherein an elevated level above a certain threshold predicts residual congestion after therapy or intervention of congestion whereas a level below a certain threshold predicts decongestion after therapy, or
      d) Correlating said level of immunoreactive analyte with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
      e) Correlating said level of immunoreactive analyte with the assessment of the decision on hospital discharge wherein an elevated level above a certain threshold means that the subject shall not be discharged and wherein a level below a certain threshold means that the subject may be discharged,
      wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6.
   A4. A method according to any of items 1 to 3, wherein said extent of congestion is expressed as congestion score, in particular a clinical congestion score.
   A5. A method of any of items 1 to 4, wherein said subject is stratified
      a) into groups of grades of congestion or,
      b) into non-responder and/or responder, and/or bad responder to therapy or intervention of congestion or,
      c) into a group with decongestion or into a group of residual congestion after therapy or intervention of congestion.
   A6. A method according to any of items 1 to 5 wherein said fragment may be selected from MR-proADM according to SEQ ID No.: 3 or mature ADM-NH₂ according to SEQ ID No.: 4.
   A7. A method according to any of items 1 to 6, wherein the level of Pro-Adrenomedullin or fragments thereof of at least 5 amino acids is determined by using a binder to Pro-Adrenomedullin or fragments thereof of at least 5 amino acids.
   A8. A method according to item 7, wherein the binder is selected from the group comprising an antibody, an antibody fragment or a non-Ig-Scaffold binding to Pro-Adrenomedullin or fragments thereof of at least 5 amino acids.
   A9. A method according to any of the preceding items, wherein said threshold is within a threshold range that is a threshold range for plasma mature ADM-NH₂ according to SEQ ID No.: 4 between 50 and 100 pg/ml and for plasma MR-proADM between 0.5 and 1.5 nmol/L, and for plasma CT-proADM between 85 and 350 pmol/L.
   A10. A method according to any of items 1 to 9, wherein said determination of Pro-Adrenomedullin or fragments thereof of at least 5 amino acids is performed more than once in one patient.
   A11. A method according to any of items 1 to 10, wherein the sample is taken at admission to a hospital or discharge of hospital.
   A12. A method according to any of items 1 to 11, wherein said monitoring is performed in order to evaluate the response of said subject to preventive and/or therapeutic measures taken.
   A13. A method according to any of items 1 to 12 in order to stratify said subjects into congestion grade groups.
   A14. A method according to any of items 1 to 13, wherein said level of Pro-Adrenomedullin or fragments thereof or said level of immunoreactive analyte is correlated with a risk of death or an adverse event in subject having acute heart failure, wherein an elevated level above a certain threshold is predictive for an enhanced risk of death or adverse events.
   A15. A method according to any of items 1 to 14, wherein said level of Pro-Adrenomedullin or fragments thereof or said level of immunoreactive analyte is used for guidance of therapy or intervention of congestion, wherein therapy or intervention is indicated if said level of Pro-Adrenomedullin or fragments is above a certain threshold and wherein-, therapy or intervention of congestion is not indicated if said level of Pro-Adrenomedullin or fragments is below a certain threshold.
   A16. A method according to any of items 1 to 15, wherein said level of Pro-Adrenomedullin or fragments thereof or said level of immunoreactive analyte is used for determining the need of therapy or intervention of congestion.
   A17. A method according to any of items 1 to 16, wherein said intervention or therapy is selected from the group comprising administration of diuretics, administration of inotropes, administration of vasodilators, ultrafiltration.
   A18. Use of Pro-Adrenomedullin or fragments thereof of at least 5 amino acids as a marker for congestion in a method for
      a) diagnosing congestion or assessing or monitoring the extent of congestion in a subject or,
      b) predicting or determining or monitoring need of therapy or intervention of congestion or predicting or determining or monitoring the success of a therapy or intervention of congestion or guiding a therapy or intervention of congestion in a subject or,
      c) predicting decongestion or residual congestion after therapy or intervention of congestion in a subject or,
      d) assessing decongestion or residual congestion after therapy or intervention of congestion in a subject or,
      e) assessing the decision on hospital discharge of a subject,
wherein said subject is a subject having acute heart failure that is either new-onset AHF or acute decompensated HF or acute decompensated chronic HF or wherein said subject is a subject having chronic heart failure with worsening signs/symptoms of chronic heart failure; and
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH2 according to SEQ ID No.: 4 or ADM-Gly according to SEQ ID No.: 5 or CT-proADM according to SEQ ID No.: 6.

### Figure Description

Figure 1:

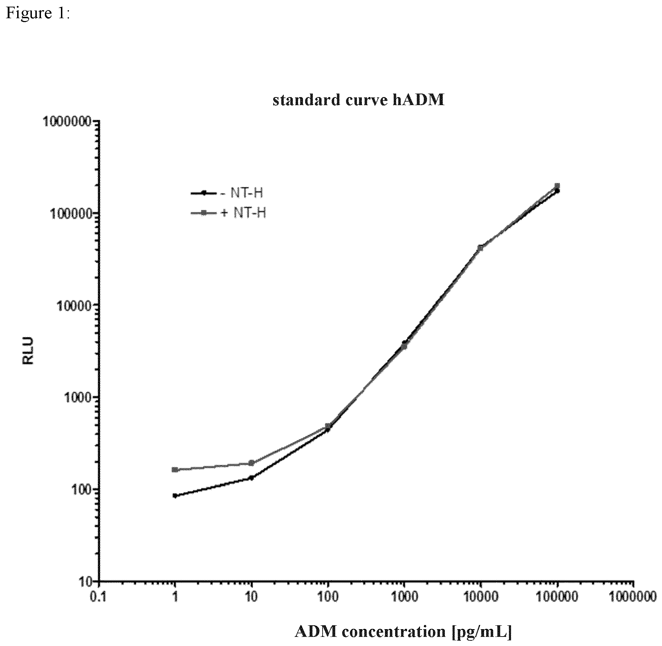

Figure 1 shows a typical bio-ADM dose/ signal curve and an bio-ADM dose signal curve in the presence of 100 µg/mL antibody NT-H.
Figure 2:
Serial measurement of bio-ADM in patients with different grades of congestion
Figure 3:
Illustration of interaction between diuretics treatment at discharge and bio-ADM (admission). Kaplan-Meier plot of treatment with and without diuretics, separately for patients with bio-ADM below or above 70 pg/mL. Diuretics may be most beneficial in those with high bio-ADM on admission.

| | |
|---|---|
| FALSE 0: | bio-ADM < 70pg/mL, not treated with diuretics |
| FALSE 1: | bio-ADM < 70pg/mL, treated with diuretics |
| TRUE 0: | bio-ADM > 70pg/mL, not treated with diuretics |
| TRUE 1: | bio-ADM > 70pg/mL, treated with diuretics |

Figure 4:
Boxplot of bio-ADM for patients with significant congestion (CCS =3) versus patients with no or mild congestion (CCS < 3, p<0.001).
Figure 5:
Boxplot of bio-ADM for patients with significant congestion (CCS =3) versus patients with no or mild congestion (CCS < 3, p<0.01).

### Examples

### Example 1

### Generation of Antibodies and determination of their affinity constants

We developed mouse monoclonal antibodies binding to the N-terminal (NT-ADM), mid-regional (MR-ADM) and C-terminal (CT-ADM) part of bio-ADM and their affinity constants were determined (Table 1).

Peptides for immunization

Peptides were supplied by JPT Peptide Technologies GmbH (Berlin, Germany). Peptides were coupled to BSA using the Sulfo-SMCC crosslinking method. The crosslinking procedure was performed according the manufacturers instructions (Thermo Fisher/ Pierce).

### Generation of murine antibodies

A Balb/c mouse was immunized with 100 µg Peptide-BSA-Conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitoneal and one intra venous injection.

Splenocytes from the immunized mouse and cells of the myeloma cell line SP2/0 were fused with 1 ml 50% polyethylene glycol for 30 s at 37°C. After washing, the cells were seeded in 96-well cell culture plates. Hybrid clones were selected by growing in HAT medium (RPMI 1640 culture medium supplemented with 20% fetal calf serum and HAT-Supplement). After two weeks the HAT medium is replaced with HT medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined (Lane, 1985. J. Immunol. Meth. 81: 223-228*;* Ziegler et al. 1996. Horm. Metab. Res. 28: 11-15).

**Table 1:**

| Antigen/Immunogen | ADM Region | Designation | Affinity constants Kd (M⁻¹) |
|---|---|---|---|
| | | | |
| YRQSMNNFQGLRSFGC | 1-16 | NT-ADM | 1,6 x 10⁹ |
| CTVQKLAHQIYQ | 21-32 | MR-ADM | 2 x 10⁹ |
| CAPRSKISPQGY-NH2 | C-42-52 | CT-ADM | 1.1 x 10⁹ |

### Monoclonal antibody production

Antibodies were produced via standard antibody production methods (Marx et al. 1997. Monoclonal Antibody Production, ATLA 25, 121) and purified via Protein A. The antibody purities were > 95% based on SDS gel electrophoresis analysis.

### Affinity Constants

To determine the affinity of the antibodies to Adrenomedullin, the kinetics of binding of Adrenomedullin to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare).

Labelling procedure (tracer)

100 µg (100 µl) of antibody (1 mg/ ml in PBS, pH 7.4,) was mixed with 10 µl Akridinium NHS-ester (1 mg/ml in acetonitrile, InVent GmbH, Germany) (EP 0 353 971) and incubated for 20 min at room temperature. Labelled CT-H was purified by Gel-filtration HPLC on Bio-Sil^{®} SEC 400-5 (Bio-Rad Laboratories, Inc., USA). The purified labeled antibody was diluted in (300 mmol/L potassiumphosphate, 100 mmol/L NaCl, 10 mmol/L Na-EDTA, 5 g/L Bovine Serum Albumin, pH 7.0). The final concentration was approx. 800.000 relative light units (RLU) of labelled compound (approx. 20 ng labeled antibody) per 200 µL. Akridiniumester chemiluminescence was measured by using an AutoLumat LB 953 (Berthold Technologies GmbH & Co. KG).

### Solid phase

Polystyrene tubes (Greiner Bio-One International AG, Austria) were coated (18 h at room temperature) with antibody (1.5 µg antibody/0.3 mL 100 mmol/L NaCl, 50 mmol/L TRIS/ HCl, pH 7.8). After blocking with 5% bovine serum albumine, the tubes were washed with PBS, pH 7.4 and vacuum dried.

### Calibrators

Synthetic human ADM (HADM) (Bachem, Switzerland) was linearily diluted using 50 mM Tris/ HCl,
250 mM NaCl, 0.2% Triton X-100, 0.5% BSA, 20 tabs/L Protease Complete Protease Inhibitor Cocktail Tablets (Roche AG); pH 7.8. Calibrators were stored at -20°C before use.

### Example 2

### Determination of the antibody combination that yields high signal/noise ratios

### ADM Immunoassay

50 µl of sample (or calibrator) was pipetted into coated tubes, after adding labeled second antibody (200 µl), the tubes were incubated for 2 h at room temperature. Unbound tracer was removed by washing 5 times (each 1 ml) with washing solution (20 mM PBS, pH 7.4, 0.1 % Triton X-100). Tube-bound chemiluminescence was measured by using the LB 953 (Berthold Technologies GmbH & Co. KG).

All antibodies were used in a sandwich immunoassay as coated tube and labeled antibody and combined in the following variations (see Table 2). Incubation was performed as described under hADM-Immunoassay. Results are given in ratio of specific signal (at 10 ng/ml ADM) /background (sample without ADM) signal.

**Table 2:**

| Signal/ noise ratio | NT-ADM tracer | MR-ADM tracer | CT-ADM tracer |
|---|---|---|---|
| NT-ADM | / | 195 | 241 |
| MR-ADM | 204 | / | 904 |
| CT-ADM | 260 | 871 | / |

Surprisingly, we found the combination of MR-ADM and CT-ADM as combination for highest signal/noise ratio.

Subsequently, we used this antibody-combination for further investigations to measure bio-ADM. We used anti-MR-ADM as solid phase antibody and anti-CT-ADM as labeled antibody. A typical dose/ signal curve is shown in Fig 1. The analytical sensitivity (average of 10 runs, ADM-free sample + 2SD) of the assay was 2 pg ADM/ml.

### Example 3

### Stability of human Adrenomedullin

Human ADM was diluted in human Citrate plasma (n=5, final concentration lOng ADM/ml) and incubated at 24 °C. At selected time points, aliquots were frozen at -20 °C. Immediately after thawing the samples hADM was quantified by using the hADM immunoassay described above.

**Table 3 shows the stability of hADM in human plasma at 24 °C.**

| Time (h) | Average ADM recovery (N=5) | Relative loss of immune reactivity | Loss of immune reactivity %/ h |
|---|---|---|---|
| 0 | 100 | / | / |
| 2 | 99,2 | 0,8 | 0,4 |
| 4 | 96,4 | 3,6 | 0,8 |
| 8 | 88,2 | 11,8 | 1,5 |
| | | | Average: 0.9%/ h |

Surprisingly, using the antibody-combinations MR-ADM and CT-ADM in a sandwich immune assay, the pre-analytical stability of the analyte is high (only 0.9%/h average loss of immune reactivity). In contrast, using other assay methods, a plasma half-life of only 22 min was reported (Hinson et al. 2000 Endocrine Reviews 21(2): 138-167). Since the time from taking sample to analysis in hospital routine is less than 2h, the used ADM detection method is suitable for routine diagnosis. It is remarkable, that any non-routine additives to samples (like aprotinin, (Ohta et al. 1999. Clin Chem 45 (2): 244-251)) are not needed to reach acceptable ADM-immune reactivity stabilities.

### Example 4

### Reproducibility of calibrator-preparations

We found a high variation of results, preparing calibrators for ADM assays (average CV 8.5%, see Table 4). This may be due to high adsorption of hADM to plastic and glass surfaces (Lewis et al. 1998. Clinical Chemistry 44 (3): 571-577). This effect was only slightly reduced by adding detergents (up to 1% Triton X 100 or 1% Tween 20), protein (up to 5% BSA) and high ionic strength (up to 1M NaCl) or combinations thereof. Surprisingly, if a surplus of anti-ADM antibody (10µg/ml) is added to the calibrator dilution buffer, the recovery and reproducibility of ADM assay calibrator-preparations was substantially improved to < 1% of inter preparation CV (Table 4).

Fortunately, the presence of N-terminal antibodies did not affect the bio-ADM-signal generated by the combination of MR- and C-terminal antibodies (Fig. 1).

**Table 4:**

| | In the presence of NT-ADM antibody (10µg/ml) | Inter preparation CV (%) | Without antibody | Inter preparation CV (%) |
|---|---|---|---|---|
| calibrator | | | | |
| 100ng/ml | 3453 s/n-r | 0.9 | 2842 s/n-r | 2.8 |
| 10ng/ml | 1946 s/n-r | 0.8 | 1050 s/n-r | 7.9 |
| 1 ng/ml | 179 s/n-r | 1.1 | 77 s/n-r | 14.8 |
| | | Average: 0.93 | | Average: 8.5 |

### Interpreparation variation of calibrators

ADM assay calibrators were prepared as described above with and without 10µg/ml of NT-ADM-antibody. Coeffients of variation are given from 5 independent preparation runs. The calibrators were measured using the ADM assay described above (s/n-r = signal to noise ratio). For all following studies, we used an ADM assay, based on calibrators, prepared in the presence of 10µg/ml of NT-ADM antibody and 10µg/ml of NT-ADM antibody as supplement in the tracer buffer.

### Example 5

### Sensitivity

The goal of assay sensitivity was to completely cover the ADM concentration of healthy subjects.

### Bio-ADM concentration in healthy subjects

Healthy subjects (n=100, average age 56 years) were measured using the bio-ADM assay. The median value was 24.7 pg/ml, the lowest value 11 pg/ml and the 99^{th} percentile 43 pg/ml. Since the assay sensitivity was 2 pg/ml, 100% of all healthy subjects were detectable using the described bio-ADM assay.

A commercial fully automated homogeneous time-resolved fluoroimmunoassay was used to measure MR-proADM in plasma (BRAHMS MR-proADM KRYPTOR; BRAHMS GmbH, Hennigsdorf, Germany) (Caruhel et al. 2009. Clin Biochem. 42 (7-8): 725-8).

### Example 6

### (PROTECT)

### Study population and measurements

The details of this study have been published (Massie et al. 2010. N Engl J Med. 363:1419-1428*.;* Weatherleyfunctioned al. 2010. J Card Fail. 16:25-35*.;* Voors et al. 2011. J Am Coll Cardiol. 57:1899-1907). In brief, 2,033 acute heart failure patients with renal function impairment (estimated creatinine clearance between 20 to 80 mL/min with Cockcroft-Gault formula) were included and randomized to rolofylline or placebo. The protocol of the PROTECT study was approved by the ethics committee at each participating center, and written informed consent was obtained from all participants.

Bio-ADM was measured from plasma collected during baseline evaluation in 1572 hospitalized AHF patients included in the PROTECT trial (all available baseline samples) using an immunoassay developed by Sphingotec GmbH (Hennigsdorf, Germany). PROTECT (stands for Placebo-controlled Randomized Study of the Selective A1 Adenosine Receptor Antagonist Rolofylline for Patients Hospitalized with Acute Decompensated Heart Failure and Volume Overload to Assess Treatment Effect on Congestion and Renal Function) was a multicenter, randomized, double-blind trial that compared rolofylline against placebo in 2033 patients hospitalized for AHF.

### Study outcomes

### Clinical congestion score

As highlighted previously, clinical surrogates have less than optimal predictive value for the detection of congestion. In this analysis, we combined three of the strongest clinical surrogates of congestion (i.e. JVP, peripheral edema and orthopnea) to enhance accuracy and developed a composite clinical congestion score (CCS) using the scheme presented below:

| Parameter | 0 | 1 | 2 | 3 |
|---|---|---|---|---|
| Peripheral edema | 0 | Ankle | Below knee | Above knee |
| Orthopnea | 0 pillow | 1 pillow | 2 pillows | 3 pillows |
| JVP | <6cm | 6-10cm | >10cm | - |

The score on each of these three parameters were then added to obtain a composite congestion score which ranged from 0 to 8. A similar scheme was previously utilized by Ambrosy et al. (Ambrosy et al. 2013. European Heart Journal 34 (11): 835-843).

The following algorithm was then utilized to grade severity of congestion:
CCS=0, No clinical congestion
CCS 1-3, Mild clinical congestion
CCS 4-5, Moderate clinical congestion
CCS ≥6, Severe clinical congestion

### Markers of decongestion

*Diuretic response:* defined as weight loss by day 4 per 40mg of diuretic dose.

*Hemoconcentration:* coded as 0 (if there is a decline or no change in hemoglobin levels by day 4 compared to baseline) or 1 (if there is an increase in hemoglobin levels by day 4 compared to baseline).

*Significant residual congestion:* defined as a CCS ≥2 based on JVP, orthopnea and edema assessments by day 7.

### Statistical analysis

Baseline clinical characteristics and biomarkers including bio-ADM were summarized by severity of clinical congestion at baseline (scheme presented above). Baseline factors independently associated with severity of clinical congestion at baseline were determined using a multivariable logistic regression model (the CCS variable was recoded as a binary outcome with two levels; 0=mild/moderate (CCS<6) and 1=severe (CCS≥6)).

Association between baseline bio-ADM levels and diuretic response (a continuous variable) was evaluated using linear regression analysis. For the hemoconcentration and significant residual congestion outcomes, binary logistic regression analysis was performed. Multivariable models were utilized to evaluate adjusted associations between bio-ADM levels and these outcomes.

### Results

In Table 5 it is demonstrated that bio-ADM concentrations increase with the severity of congestion.

**Table 5: Baseline clinical variables and biomarkers by severity of clinical congestion at baseline**

| | **Mild clinical congestion N=212** | **Moderate clinical congestion N=528** | **Severe clinical congestion N=667** | **P-trend** |
|---|---|---|---|---|
| Male sex, % (N) | 65.6 (139) | 68.4 (361) | 65.4 (436) | 0.656 |
| Age (yrs) | 71.5±11.7 | 71.1±11.3 | 70.8±10.7 | 0.427 |
| **BMI (Kg/m2)** | **26.5±4.4** | **27.8±5.3** | **29.9±6.4** | **<0.001** |
| LVEF (%) | 32.1±13.4 | 33.2±13.4 | 32.2±13.4 | 0.808 |
| SBP (mmHg) | 124.2±17.9 | 125.2±17.4 | 124.3±17.3 | 0.773 |
| DBP (mmHg) | 72.9±12.2 | 73.3±11.4 | 74.1±11.6 | 0.118 |
| Heart Rate (bpm) | 77.6±13.6 | 79.1±15.4 | 81±15.9 | 0.002 |
| Respiratory rate (per min) | 20 [18-22] | 20 [18-24] | 21 [18-24] | 0.001 |

| Orthopnea, % (N) | | | | |
|---|---|---|---|---|
| None | 17.9 (38) | 4 (21) | 0 (0) | ref. |
| One pillow (10 cm) | 36.8 (78) | 14.8 (78) | 2.5 (17) | <0.001 |
| Two pillows (20 cm) | 33.0 (70) | 56.6 (299) | 31.8 (212) | <0.001 |
| > 30 degrees | 12.3 (26) | 24.6 (130) | 65.7 (438) | <0.001 |

| Pulmonary rates, % (N) | | | | |
|---|---|---|---|---|
| < 1/3 | 25.5 (54) | 33.5 (177) | 28.9 (193) | ref. |
| 1/3 - 2/3 | 57.1 (121) | 51.5 (272) | 48.9 (326) | 0.333 |
| >2/3 | 17.5 (37) | 15 (79) | 22.2 (148) | 0.101 |

| Edema, % (N) | | | | |
|---|---|---|---|---|
| 0 | 56.6 (120) | 15.3 (81) | 0(0) | ref. |
| 1+ | 29.2 (62) | 33.1 (175) | 5.7 (38) | <0.001 |
| 2+ | 13.2 (28) | 43.2 (228) | 47.2 (315) | <0.001 |
| 3+ | 0.9 (2) | 8.3 (44) | 47.1 (314) | <0.001 |

| JVP, % (N) | | | | |
|---|---|---|---|---|
| < 6 cm | 42.5 (90) | 12.3 (65) | 24 (16) | ref. |
| 6 - 10 cm | 52.4 (111) | 62.3 (329) | 34.9 (233) | <0.001 |
| > 10 cm | 5.2 (11) | 25.4 (134) | 62.7 (418) | <0.001 |

| NYHA, % (N) | | | | |
|---|---|---|---|---|
| I | 0.9 (2) | 1.5 (8) | 0.7 (5) | 0.784 |
| II | 241 (51) | 15 (79) | 144 (96) | 0.779 |
| III | 50.5 (107) | 50.4 (266) | 47.4 (316) | 0.668 |
| IV | 14.6 (31) | 28.6 (151) | 34.6 (231) | 0.494 |

| **Medical history** | | | | |
|---|---|---|---|---|
| COPD, % (N) | 15.1 (32) | 20.1 (106) | 22 (146) | 0.039 |
| Stroke, % (N) | 8 (17) | 8.3 (44) | 109 (73) | 0.111 |
| Peripheral vascular disease, % (N) | 12.3 (26) | 11.6 (61) | 9.3 (62) | 0.145 |
| Hypertension, % (N) | 78.8 (167) | 77.7 (410) | 82.6 (551) | 0.075 |
| Diabetes mellitus, % (N) | 38.7 (82) | 44.1 (233) | 48.1 (321) | 0.013 |
| Hypercholesterolemia, % (N) | 58 (123) | 50 (264) | 51.1 (340) | 0.189 |
| Myocardial infarction, % (N) | 52.8 (112) | 50.1 (264) | 49.4 (328) | 0.424 |
| Angina, % (N) | 20.8 (44) | 20.7 (109) | 24 (160) | 0.187 |
| Ischaemic heart disease, % (N) | 68.9 (146) | 70.4 (371) | 71.4 (475) | 0.468 |
| Atrial fibrillation, % (N) | 44.3 (94) | 53.0 (279) | 59.8 (396) | <0.001 |
| Past HF hospitalization, % (N) | 41.5 (88) | 47.2 (249) | 53.4 (356) | 0.001 |
| PCI, % (N) | 34.0 (71) | 26.5 (139) | 23.3 (154) | 0.003 |
| CABG, % (N) | 26.3 (55) | 20.6 (108) | 22.5 (149) | 0.514 |
| Pacemaker, % (N) | 9.9 (21) | 11 (58) | 11.4 (76) | 0.556 |

| **Medication during hospital admission** | | | | |
|---|---|---|---|---|
| ACEI/ARB, % (N) | 78.3 (166) | 75.7 (399) | 75.6 (504) | 0.492 |
| Beta-blocker, % (N) | 82.1 (174) | 75.1 (396) | 75.1 (501) | 0.089 |
| MRA, % (N) | 41.5 (88) | 44.8 (236) | 47.5 (317) | 0.110 |
| Digoxin, % (N) | 20.3 (43) | 30.6 (161) | 31.3 (209) | 0.009 |

| **Biomarkers** | | | | |
|---|---|---|---|---|
| **Albumin (g/dL)** | **4.0 [3.7-4.3]** | **3.9 [3.6-4.2]** | **3.8 [3.5-4.1]** | **<0.001** |
| ALT(U/L) | 20 [14-32] | 21 [15-32] | 21 [15-30] | 0.299 |
| AST (U/L) | 23 [17-31] | 24 [19-33] | 25 [20-33] | 0.210 |
| Bicarbonate (mEq/L) | 23.6±3.4 | 24.2±3.6 | 24±3.9 | 0.485 |
| BUN (mg/dL) | 29 [22-42] | 28 [21-40] | 31 [22-42] | 0.057 |
| Chloride (mEq/L) | 102 [99-105] | 101 [98-104] | 101 [98-104] | 0.085 |
| Creatinine (mg/dL) | 1.4 [1.1-1.8] | 1.4 [1.1-1.8] | 1.4 [1.1-1.8] | 0.59 |
| **Total cholesterol (mg/dL)** | **154.5 [122-183.5]** | **146.5 [123-173.8]** | **134 [110-164]** | **<0.001** |
| Glucose (mg/dL) | 126 [104.5-155] | 126 [101-168] | 128 [103-162] | 0.663 |
| Hemogblobin (g/dL) | 12.6±1.8 | 12.5±1.8 | 12.5±1.9 | 0.716 |
| Platelet count (*10^9/1) | 221 [180-278] | 218 [170-268] | 211 [168-260] | 0.020 |
| Pottasium (mmol/L | 4.3±0.6 | 4.3±0.6 | 4.3±0.6 | 0.723 |
| RBC count (*10^9/1) | 4.2±0.6 | 4.2±0.6 | 4.2±0.6 | 0.200 |
| Sodium (mmol/L) | 140 [137-142] | 140 [138-142] | 140 [137-142] | 0.139 |
| Triglycerides (mmol/L) | 96 [66-132.5] | 89 [65-130] | 83 [64-112] | 0.003 |
| Uric acid (mg/dL) | 8.5±2.5 | 8.8±2.6 | 9.1±2.5 | 0.003 |
| WBC (*10^9/1 | 7.9 [6.5-10] | 7.3 [6.2-9.1] | 7.3 [5.9-9] | 0.003 |
| Hematocrite (%) | 39.5±5.4 | 39.4±5.6 | 40.1±5.9 | 0.09 |
| **BNP (pg/ml)** | **406.8 [255.8-648.2]** | **437.4 [247.9-797.1]** | **487.9 [274.8-874]** | **0.002** |
| cTnI (pg/mL) | 12.0 [5.8-38.2] | 9.8 [5.2-20.1] | 10.8 [5.8-22.7] | 0.046 |
| **bio-ADM (pg/mL)** | **30.0 [20.2-46.1]** | **37.4 [22.5-66.8]** | **55.4 [31.4-102.6]** | **<0.001** |

Furtheron, it was demonstrated by multivariable logistic regression that bio-ADM is an independent predictor of the severity of congestion and the strongest among all other available variables (table 6).

**Table 6: Baseline factors independently associated with severity of baseline clinical congestion in a multivariable logistic regression model (severe versus mild/moderate)**

| **Variable** | **Odds ratio [95% CI]** | **P-value** |
|---|---|---|
| Past HF hospitalization | 1.40 | 0.004 |
| | [1.11-1.75] | |
| BMI | 1.06 | <0.001 |
| | [1.04-1.08] | |
| Serum albumin | 0.56 | <0.001 |
| | [0.43-0.74] | |
| bio-ADM, log | 1.58 | <0.001 |
| | [1.38-1.82] | |

**Area under the curve (AUC) of the whole model=0.69, Individual AUCs: bio-ADM=0.66, BMI=0.61, serum albumin=0.58, past HF hospitalization=0.54**

| **Main message from tables 5 & 6** |
|---|
| **There are very few baseline clinical variables or biomarkers associated with severity of clinical congestion at baseline and bio-ADM appears to be, by far, the strongest.** |

Furtheron it was analyzed whether bio-ADM would predict decongestion. In table 7 it is demonstrated that bio-ADM is an independent predictor of significant residual congestion by day 7.

**Table 7: Association between baseline bio-ADM levels and markers of decongestion**

| **Marker of decongestion** | **Unadjusted** | | **Adjusted** | |
|---|---|---|---|---|
| | Estimate [95% CI] | P-value | Estimate [95% CI] | P-value |
| Significant residual congestion by day 7* | 1.98 [1.70-2.32] | <0.001 | 1.30 [1.10-1.60]** | 0.009 |
| Diuretic response by day 4 | 0.02 [-0.02-0.06] | 0.350 | - | - |
| Hemoconcentration by day 4 | 0.82 [0.71-0.94] | 0.004 | 0.90 [0.78-1.04]^{#} | 0.160 |

| | | | | |
|---|---|---|---|---|
| ^{∗} defined as composite congestion score >2 by day 7 ^{∗∗}adjusted for baseline variables including orthopnea, JVP, peripheral edema, history of PCI, pacemaker, ACEI/ARB use, BMI, DBP, BUN, hematocrit and BNP ^{#}adjusted for baseline clinical congestion score (NB: there is ∼30% missingness in hemoconcentration data) | | | | |

| **Main message from table 7** |
|---|
| **Baseline levels of bio-ADM independently predict significant residual congestion by day 7.** |

As to be expected for a marker of congestion, bio-ADM concentrations were higher the more diuretics were used for therapy (tables 8 and 9)

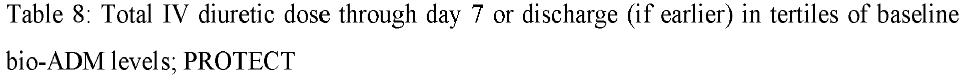

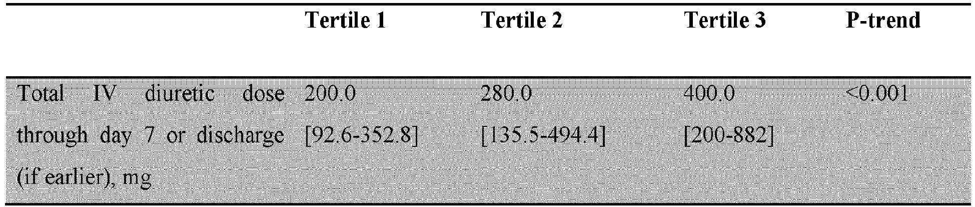

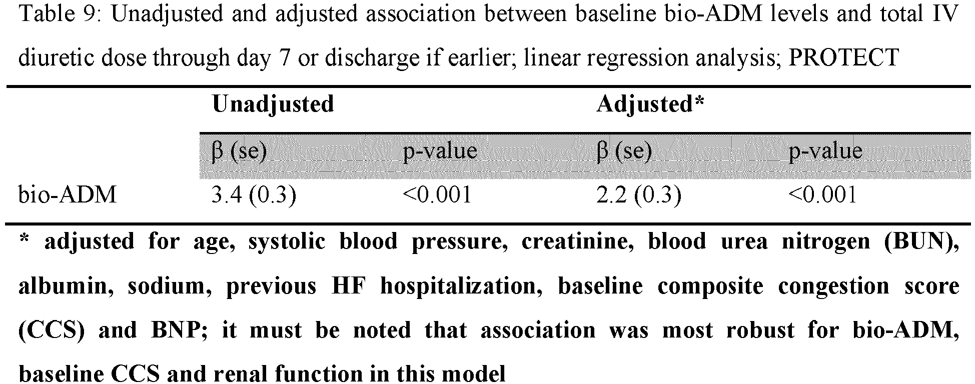

In the same sample set also MR-proADM was determined. Baseline characteristics by tertiles of MR-proADM are shown in table 10: Similar to bio-ADM, increasing levels of MR-proADM were associated with increasing extent of edema.

**Table 10: Baseline characteristics by tertiles of MR-proADM**

| | | | | |
|---|---|---|---|---|
| | | | | |

| Variable | Tertile 1 | Tertile 2 | Tertile 3 | P-trend |
|---|---|---|---|---|
| | N=516 | N=515 | N=516 | |
| MR-proADM (nmol/l) | 0.24 [0.14-0.29] | 0.55 [0.47-0.67] | 1.25 [0.94-1.88] | |
| Sex | 65.9 (340) | 64.3 (331) | 69.4 (358) | 0.235 |
| Age (yrs) | 70.1±11.1 | 71.3±10.9 | 71.3±11.4 | 0.073 |
| BMI (Kg/m2) | 28±5.5 | 28.9±5.9 | 29.5±6.5 | <0.001 |
| LVEF (%) | 32.9±13.4 | 33.5±12.3 | 31.3±13.8 | 0.178 |
| SBP (mmHg) | 126.3±17.5 | 125.9±17.1 | 122.2±17.4 | <0.001 |
| DBP (mmHg) | 74.1±12.1 | 74.3±11.6 | 72.6±11.7 | 0.033 |
| Heart Rate (bpm) | 78.7±14.4 | 79.8±15.4 | 81.2±16.5 | 0,008 |
| Respiratory rate (per min) | 20 [18-24] | 20 [18-24] | 22 [18.8-24] | 0,698 |

| Orthopnea | | | | |
|---|---|---|---|---|
| None | 3.5 (18) | 4.3 (22) | 3.3 (17) | ref. |
| One pillow | 12.8 (66) | 10.5 (54) | 13.2 (68) | 0,819 |
| Two pillows | 43.4 (224) | 43.3 (223) | 34.5 (178) | 0,619 |
| >30 degrees | 39.1 (202) | 41.2 (212) | 48.1 (248) | 0,439 |

| Rales | | | | |
|---|---|---|---|---|
| None | 8.3 (43) | 7.6(39) | 11.2(58) | ref. |
| <1/3 | 24.6 (127) | 29.1 (150) | 32.6 (168) | 0,849 |
| 1/3-2/3 | 58.3 (301) | 53 (273) | 45.7 (236) | 0,012 |
| >2/3 | 8.5 (44) | 10.3 (53) | 10.3 (53) | 0,622 |

| Edema | | | | |
|---|---|---|---|---|
| 0 | 16.3 (84) | 14.8 (76) | 12.2 (63) | ref. |
| 1+ | 24.8 (128) | 17.5 (90) | 15.3 (79) | 0,324 |
| 2+ | 39.7 (205) | 40 (206) | 40.5 (209) | 0,112 |
| 3+ | 19.2 (99) | 27.8 (143) | 31.8 (164) | <0.001 |

| JVP | | | | |
|---|---|---|---|---|
| <6 cm | 11 (57) | 9.3 (48) | 126 (65) | ref. |
| 6-10 cm | 45.3 (234) | 42.3 (218) | 40.3 (208) | 0,22 |
| >10 cm | 34.7 (179) | 37.7 (194) | 37.4 (193) | 0,755 |

| NYHA | | | | |
|---|---|---|---|---|
| I | 1.7 (9) | 0.8 (4) | 0.6 (3) | 0,024 |
| II | 17.4 (90) | 17.5 (90) | 13.8 (71) | 0,062 |
| III | 50.6 (261) | 49.3 (254) | 44.8 (231) | 0,07 |
| IV | 25.6 (132) | 29.1 (150) | 33.7 (174) | 0,114 |
| COPD | 18.6 (96) | 19.2 (99) | 21.9 (113) | 0,185 |
| Stroke | 8.7 (45) | 8 (41) | 11.2 (58) | 0,164 |
| Peripheral vascular disease | 10.1 (52) | 11.9(61) | 11.9(61) | 0,37 |
| Hypertension | 79.1 (408) | 83.3 (429) | 78.7 (406) | 0,876 |
| Diabetes mellitus | 44.6 (230) | 46 (237) | 47.5 (245) | 0,349 |
| Hypercholestrolemia | 53.8 (277) | 52.6 (271) | 46.9 (242) | 0,027 |
| Myocardial infarction | 49.2 (252) | 50.5 (260) | 50.4 (260) | 0,708 |
| Angina | 22.5 (116) | 21.2 (109) | 23.9 (123) | 0,59 |
| Ischaemic heart disease | 69.2 (355) | 72.8 (375) | 69.6 (359) | 0,898 |
| Atrial fibrillation | 48 (246) | 56.5 (290) | 58.8 (302) | 0,001 |
| Past HF hospitalization | 44.8 (231) | 48.9 (252) | 53.3 (275) | 0,006 |
| PCI | 27.5 (140) | 25.6 (131) | 24.9 (127) | 0,344 |
| CABG | 21.4 (109) | 21.9 (112) | 23.2 (119) | 0,472 |
| Pacemaker | 8(41) | 13.4 (69) | 13 (67) | 0,012 |
| ACEI/ARB | 77.7 (401) | 78.6 (404) | 71.5 (369) | 0,02 |
| Beta-blocker | 79.1 (408) | 74.5 (383) | 72.5 (374) | 0,014 |
| MRA | 44.6 (230) | 45.9 (236) | 45 (232) | 0,901 |
| Digoxin | 28.7 (148) | 30.7 (158) | 27.3 (141) | 0,631 |

| **Standard lab parameters** | | | | |
|---|---|---|---|---|
| Albumin (g/dL) | 3.9 [3.6-4.2] | 3.9 [3.6-4.2] | 3.8 [3.5-4] | <0.001 |
| ALT(U/L) | 21 [15-30] | 20 [15-31] | 21 [15-34] | 0,003 |
| AST (U/L) | 24 [18-31] | 25 [19-32.8] | 26 [19-37] | 0,003 |
| Bicarbonate (mEq/L) | 24.5±3.6 | 24.4±3.7 | 23.3±3.8 | <0.001 |
| BUN (mg/dL) | 25 [19-33] | 29 [22-38.2] | 38 [28-51] | <0.001 |
| Chloride (mEq/L) | 101 [99-104] | 101 [99-104] | 101 [97-104] | 0,023 |
| Creatinine (mg/dL) | 1.2 [1-1.5] | 1.4 [1.1-1.7] | 1.6 [1.3-2.1] | <0.001 |
| Total cholesterol (mg/dL) | 151 [126-181.2] | 146 [119-174] | 129 [107.5-159.5] | <0.001 |
| Glucose (mg/dL) | 129.5 [104-171.5] | 126 [103-164] | 128 [101-155] | 0,013 |
| Hemogblobin (g/dL) | 12.8±1.9 | 12.6±1.7 | 12.2±1.9 | <0.001 |
| Platelet count (*10^9/1) | 227 [180-277] | 214.5 [172-266.5] | 207 [161.5-263] | 0,011 |
| Pottasium (mmol/L | 4.3±0.6 | 4.2±0.6 | 4.3±0.6 | 0,22 |
| RBC count (*10^9/l) | 4.3±0.6 | 4.2±0.6 | 4.1±0.6 | <0.001 |
| Sodium (mmol/L) | 140 [138-142] | 140 [138-143] | 139 [136-142] | <0.001 |
| Triglycerides (mmol/L) | 101 [71-138.5] | 83 [63-118] | 81 [61-107] | <0.001 |
| Uric acid (mg/dL) | 8.3±2.4 | 8.9±2.4 | 9.7±2.8 | <0.001 |
| WBC (* 10^9/l | 7.7 [6.3-9.2] | 7.3 [6.1-9.1] | 7.2 [5.9-9.3] | 0,591 |
| Hematocrite (%) | 40.4±5.8 | 40±5.5 | 38.9±5.9 | <0.001 |

| **Biomarkers** | | | | |
|---|---|---|---|---|
| IL-6 (pg/ml) | 9.1 [5.3-16.5] | 10.8 [6.4-19.9] | 14.3 [8.8-26] | 0,037 |
| CRP (ng/ml) | 12566.3 [6334.9-25975.8] | 13306.5 [7168.3-27844.1] | 16044.9 [8412.3-29966.3] | 0,015 |
| D-Dimer (ng/ml) | 134 [90.6-301] | 155.5 [90.6-340.8] | 180.3 [90.6-374.7] | 0,017 |
| PIGR (ng/ml) | 292.2 [197.5-500.4] | 363 [263.5-531.6] | 578.8 [371.4-999] | <0.001 |
| Pentraxin-3 (ng/ml) | 3.4 [2.2-6] | 3.9 [2.8-6] | 5.5 [3.6-8.9] | <0.001 |
| GDF-15 (ng/ml) | 3.6 [2.5-5.8] | 4 [3-6.1] | 6.3 [4.3-6.3] | <0.001 |
| RAGE (ng/ml) | 4.7 [3.4-6.5] | 4.9 [3.6-6.4] | 5.4 [4-7.4] | <0.001 |
| TNF-R1a (ng/ml) | 2.6 [1.7-4] | 2.9 [2.2-3.9] | 4.4 [3.2-5.9] | <0.001 |
| PCT (pg/ml) | 14 [8-31] | 20 [11-41] | 35.5 [18-78] | <0.001 |
| Myeloperoxidase (ng/ml) | 33.9 [17.7-78.5] | 30.9 [18.7-60] | 32.8 [17-66.2] | 0,209 |
| Syndecan-1 (ng/ml) | 7.7 [6.4-9.5] | 8.1 [6.9-9.4] | 9.6 [8.1-11.6] | <0.001 |
| Periostin (ng/ml) | 4 [2.3-6.8] | 5.8 [3.5-8.8] | 7.3 [4.3-11.2] | <0.001 |
| Galectin-3 (ng/ml) | 32 [25.2-43.1] | 34 [26.5-44.1] | 42.7 [32.5-55.9] | <0.001 |
| Osteopontin (ng/ml) | 98.3 [66.3-149.9] | 106.4 [74.2-150.3] | 133.8 [100.7-190.8] | <0.001 |
| ET1 (pg/ml) | 5.7 [4.2-7.6] | 6.7 [5.1-9] | 7.9 [6.1-11] | <0.001 |
| BNP (pg/ml) | 310.8 [189.8-548.6] | 477.2 [262.6-783.3] | 630.1 [341.3-1063] | <0.001 |
| NT-proCNP (pg/ml) | 34 [22-48] | 42 [31-58] | 53 [38-74.2] | <0.001 |
| ST-2 (ng/ml) | 1.8 [0.9-5.6] | 2.8 [1-5.7] | 6.1 [2.7-10.9] | <0.001 |
| VEGFR (ng/ml) | 0.3 [0.2-0.4] | 0.4 [0.2-0.5] | 0.5 [0.3-0.8] | <0.001 |
| Angiogenin (ng/ml) | 1940 [1229.1-2950.6] | 1979.3 [1388.1-3093.5] | 1675.8 [1178.7-2552.6] | 0,007 |
| Mesothelin (ng/ml) | 81.6 [65.6-97] | 83.6 [73.6-94.2] | 94.5 [83.7-108.1] | <0.001 |
| Neuropilin SAND (ng/ml) | 10.6 [6.6-15.8] | 11.6 [8.1-16.8] | 14.8 [10.8-20.4] | <0.001 |
| Troy (pg/ml) | 72 [49-110] | 84 [64-115] | 114 [83-153] | <0.001 |
| bio-ADM | 28 [17.2-53.5] | 42.9 [27.1-74.2] | 70.9 [40.3-120.3] | <0.001 |
| LTBR (ng/ml) | 0.3 [0.2-0.5] | 0.4 [0.3-0.5] | 0.5 [0.4-0.7] | <0.001 |
| ESAM (ng/ml) | 59.2 [53.4-68] | 60.2 [55.6-65.8] | 66.3 [60.5-71.7] | <0.001 |
| KIM-1(pg/ml) | 273.4 [169.1-484.6] | 296 [181.3-480.4] | 316.8 [199.3-492.9] | 0,485 |
| NGAL(ng/ml) | 64.8 [44.2-98.6] | 81.8 [54.2-123] | 107.2 [70.4-175.2] | <0.001 |
| PSAP-B(ng/ml) | 34 [25.3-49.7] | 36.9 [27.9-47.8] | 45.4 [34.1-61.1] | <0.001 |
| WAP4C(ng/ml) | 18.2 [8.7-38.8] | 22.6 [13.9-38.5] | 45.8 [26.9-67.8] | <0.001 |
| cTnI(pg/ml) | 9.3 [5.1-19.5] | 10.9 [5.6-21.2] | 12.5 [6.3-27.2] | 0,078 |

### Example 7

### (BIO STAT)

Additional analyses were performed in the BIOSTAT Study (BIOlogy Study to TAilored Treatment in Chronic Heart Failure). The study has been described in detail (WWW.BIOSTAT-CHF.EU; Voors et al. 2016. Eur J Heart Fail. Jun;18(6):716-26)*.* The BIOlogy Study to TAilored Treatment in Chronic Heart Failure (BIOSTAT-CHF) included 2516 patients with worsening signs and/or symptoms of heart failure from 11 European countries, who were considered to be on suboptimal medical treatment. Another 1738 patients from Scotland were included in a validation cohort. Overall, both patient cohorts were well matched. The majority of patients were hospitalized for acute heart failure, and the remainder presented with worsening signs and/or symptoms of heart failure at outpatient clinics. Approximately half of the patients were in New York Heart Association class III, and 7% vs 34% of patients of the index vs validation cohort had heart failure with preserved ejection fraction. According to study design, all patients used diuretics, but owing to the inclusion criteria of both cohorts, patients were not on optimal, evidence-based medical therapy. In the follow-up phase, uptitration to guideline-recommended doses was encouraged.

### Study population

Patients fulfilled the following inclusion criteria:
- aged ≥18 years with symptoms of new-onset or worsening heart failure,
- have objective evidence of cardiac dysfunction documented either by,
- left ventricular ejection fraction of ≤40% OR,
- plasma concentrations of BNP and/or N-terminal pro-brain natriuretic peptide (NT-proBNP) >400 pg/mL or >2000 pg/ml, respectively,
- be treated with either oral or intravenous furosemide ≥40 mg/day or equivalent at the time of inclusion,
- not previously treated with evidence-based therapies [angiotensin-converting enzyme (ACE) inhibitors/angiotensin receptor antagonists (ARBs) and beta-blockers] or receiving ≤50% of target doses of these drugs at the time of inclusion,
- be anticipated to be initiated or uptitrated with ACE inhibitors/ARBs and/or beta-blockers by the treating physician.

Patients were enrolled as inpatients or from outpatient clinics. Approximately 2/3 was hospitalized and 1/3 were seen in the outpatient setting.

A subset of patients comprising all types of patients included in the trial (n=1806), for which biomarker measurements at baseline were available, was analyzed in the present invention. Similar to the PROTECT study (example 6) also in the BIOSTAT study increasing levels of bio-ADM were correlated with increasing severity of edema (table 11).

**Table 11: Baseline clinical variables and standard biomarkers by severity of peripheral edema; BIOSTAT**

| | **None** | **Ankle** | **Below knee** | **Above knee** | **P-trend** |
|---|---|---|---|---|---|
| | N =734 | N =532 | N =408 | N =132 | |
| Age (yrs) | 68 [59.7-76.2] | 70.7 [62.6-78.6] | 72.4 [64.2-78.8] | 74.3 [64.6-80.4] | <0.001 |
| BMI (Kg/m2) | 26.5 [23.9-29.4] | 27.1 [23.9-30.4] | 28.7 [24.8-33.8] | 30.5 [26.2-34.9] | <0.001 |
| LVEF (%) | 30 [25-35] | 30 [25-37] | 30 [23-38] | 35 [25-43.8] | 0.002 |
| DBP (mmHg) | 75.8±12.9 | 74.2±12.8 | 74.3±14.6 | 72.5±13 | 0.004 |
| Heart Rate (bpm) | 75 [65-85] | 78.5 [68.2-90] | 80 [70-92] | 80 [70-92.2] | <0.001 |
| Orthopnea | 21.5 (158) | 40.4 (215) | 50.7 (207) | 61.4 (81) | <0.001 |
| Rales >1/3 lung fields | 5.6 (41) | 10.7 (57) | 16.4(67) | 28.8 (38) | <0.001 |
| Elevated JVP | 11.2 (82) | 29.7 (158) | 38 (155) | 47(62) | <0.001 |
| NYHA | | | | | |
| I | 2.7 (20) | 0.8 (4) | 1 (4) | 15.2 (20) | ref. |
| II | 48.2 (354) | 31 (165) | 14.7 (60) | 57.6 (76) | 0.402 |
| III | 39.2 (288) | 53.2 (283) | 62.7 (256) | 21.2 (28) | <0.001 |
| IV | 8.2 (60) | 13.5 (72) | 18.1 (74) | 6.1 (NA) | <0.001 |
| Hepatomegally | 8 (59) | 16.2 (86) | 22.3 (91) | 27.3 (36) | <0.001 |

| Medical history | | | | | |
|---|---|---|---|---|---|
| Diabetes mellitus | 27.1 (199) | 32.7 (174) | 43.6 (178) | 36.4 (48) | <0.001 |
| Atrial fibrillation | 38.6 (283) | 49.1 (261) | 56.4 (230) | 55.3 (73) | <0.001 |
| ACEI/ARB | 73.8 (542) | 74.6 (397) | 66.7 (272) | 57.6 (76) | <0.001 |
| Beta-blocker | 86.8 (637) | 83.1 (442) | 78.2 (319) | 75 (99) | <0.001 |

| Biomarkers | | | | | |
|---|---|---|---|---|---|
| ALBUMIN1 (g/L) | 33 [28-39] | 32 [27-37.5] | 31 [26-35] | 30 [24.2-33.8] | <0.001 |
| AST (U/L) | 24 [19-32] | 26 [19-36] | 28 [21-37] | 29 [24-40.8] | 0.003 |
| Gamma-GT (U/L) | 39.7 [24-79.5] | 61 [30-107.8] | 69 [40-142.3] | 98.5 [55-174.5] | <0.001 |
| BUN (mmol/L) | 10.1 [7.1-16.5] | 12.0 [7.8-19.3] | 11.9 [8.3-19.9] | 11.3 [7.4-18.4] | 0.001 |
| Total cholesterol (mmol/L) | 4.4 [3.7-5.2] | 4 [3.3-4.9] | 3.8 [3.1-4.5] | 3.1 [2.8-3.7] | <0.001 |
| Hemogblobin (g/dL) | 13.5±1.8 | 13±1.9 | 13.1±1.9 | 12.4±2.1 | <0.001 |
| Triglycerides (mmol/L) | 1.3 [1-1.8] | 1.2 [0.9-1.5] | 1.1 [0.9-1.5] | 0.9 [0.8-1.2] | <0.001 |
| Hematocrit (%) | 40.6±5.1 | 39.7±5.4 | 39.8±5.6 | 38.3±5.8 | <0.001 |
| **BNP (pg/ml)** | **489.1 [225-812]** | **663.8 [322-1192]** | **792.5 [487-1471]** | **759.0 [557-2023]** | **0.02** |
| **bio-ADM (pg/mL)** | **26.1 [19.2-37.8]** | **36.7 [24.6-54.4]** | **52.8 [34.4-86.7]** | **82.8 [49.4-141.2]** | **<0.001** |

In the same sample set also MR-proADM was determined. Baseline characteristics by tertiles of MR-proADM are shown in table 12: Similar to bio-ADM, increasing levels of MR-proADM were associated with increasing extent of edema.

**Table 12: Baseline characteristics by tertiles of MR-proADM**

| | | | | |
|---|---|---|---|---|
| | | | | |

| Variable | Tertile 1 | Tertile 2 | Tertile 3 | P-trend |
|---|---|---|---|---|
| N= | 704 | 703 | 703 | |
| MR-proADM (nmol/L) | 0.3 [0.2-0.3] | 0.5 [0.4-0.6] | 1 [0.8-1.5] | <0.001 |
| Male sex | 74.6 (525) | 72.1 (507) | 73 (513) | 0,498 |
| Age (yrs) | 65.3 [57-73.8] | 71.2 [62.9-78.4] | 74.3 [65.2-80.4] | <0.001 |
| BMI (Kg/m2) | 26.8 [23.9-30.4] | 27.3 [24.2-30.9] | 27.1 [24.1-30.7] | 0,051 |
| LVEF (%) | 30 [25-35] | 30 [25-37.2] | 30 [25-38] | 0,297 |
| SBP (mmHg) | 126.3±20.8 | 125.8±23.4 | 121.2±21.1 | <0.001 |
| DBP (mmHg) | 76.4±13.3 | 75.2±13.3 | 72.3±13 | <0.001 |
| Heart Rate (bpm) | 75 [65-86] | 77 [68-90] | 77 [67-90] | 0,015 |
| Orthopnea present | 23 (162) | 35.8 (252) | 45.1 (317) | <0.001 |
| Rales >1/3 lung fields | 6.4 (45) | 11.9 (84) | 12.9 (91) | 0,074 |

| Edema | | | | |
|---|---|---|---|---|
| 0 | 45 (317) | 32.9 (231) | 23.2 (163) | ref. |
| 1+ | 20.5 (144) | 26.3 (185) | 26.5 (186) | <0.001 |
| 2+ | 11.9 (84) | 17.2 (121) | 27.0 (190) | <0.001 |
| 3+ | 2.1 (15) | 4.8 (34) | 11.0 (77) | <0.001 |
| Elevated JVP | 14.9 (105) | 20.9 (147) | 32.4 (228) | <0.001 |

| NYHA | | | | |
|---|---|---|---|---|
| I | 2.7 (19) | 2.6 (18) | 1 (7) | ref. |
| II | 46 (324) | 37.7 (265) | 21.5 (151) | 0,751 |
| III | 40.1 (282) | 48.1 (338) | 55.3 (389) | 0,002 |
| IV | 7.8 (55) | 9.7 (68) | 18.8 (132) | <0.001 |
| Hepatomegally | 9.7 (68) | 12.8 (90) | 19.8 (139) | <0.001 |
| S3 | 9.7 (68) | 9.7 (68) | 9.5 (67) | 0,935 |

| **Medical history** | | | | |
|---|---|---|---|---|
| COPD | 15.1 (106) | 17.4 (122) | 18.9 (133) | 0,054 |
| Stroke | 7.1 (50) | 10 (70) | 11.5 (81) | 0,005 |
| Peripheral vascular disease | 8.5 (60) | 12.5 (88) | 12.5 (88) | 0,017 |
| Hypertension | 60.8 (428) | 64.3 (452) | 61.3 (431) | 0,842 |
| Diabetes mellitus | 25.6 (180) | 35.3 (248) | 36.7 (258) | <0.001 |
| Myocardial infarction | 29.7 (209) | 39.5 (278) | 42.1 (296) | <0.001 |
| Atrial fibrillation | 37.1 (261) | 46.4 (326) | 53.5 (376) | <0.001 |
| Past HF hospitalization | 27.8 (196) | 30.4 (214) | 34.3 (241) | 0,009 |
| PCI | 17 (120) | 21.2 (149) | 23.6 (166) | 0,002 |
| CABG | 12.2 (86) | 17.8 (125) | 21.8 (153) | <0.001 |
| ACEI/ARB | 75.7 (533) | 73.1 (514) | 66.4 (467) | <0.001 |
| Beta-blocker | 84.9 (598) | 83.5 (587) | 80.1 (563) | 0,016 |

| **Standard lab parametrs** | | | | |
|---|---|---|---|---|
| Albumin (g/L) | 34 [29-39] | 33 [27-37] | 30 [24-36] | <0.001 |
| ALT(U/L) | 27 [18-40] | 25 [17-39] | 23 [15-35] | 0,452 |
| AST (U/L) | 24 [20-33] | 25.9 [18-35] | 27 [20-36] | 0,087 |
| Gamma-GT (U/L) | 40.8 [25-84] | 52 [28-99] | 76 [32.5-132.4] | <0.001 |
| Alkaline phosphatase (ug/L) | 80 [63-114.8] | 83 [65-111] | 91 [69-123.5] | 0,087 |
| BUN (mmol/L) | 8.9 [6.4-13.3] | 11 [7.4-16.2] | 15 [9.5-25] | <0.001 |
| Creatinine (mg/dL) | 89.3 [75.8-106.1] | 100 [84.6-123.4] | 124.6 [98-167] | 0,61 |
| Total cholesterol (mmol/L) | 4.5 [3.8-5.5] | 4.2 [3.4-4.9] | 3.6 [3-4.5] | <0.001 |
| Glucose (mg/dL) | 6 [5.3-7.5] | 6.4 [5.4-8.5] | 6.5 [5.4-7.9] | 0,22 |
| Hemogblobin (g/dL) | 13.7±1.7 | 13.3±1.8 | 12.5±1.9 | <0.001 |
| Platelet count (* 10^9/1) | 221 [183-264] | 220 [173-263] | 208 [161-258] | 0,001 |
| Pottasium (mmol/L | 4.3 [4-4.6] | 4.2 [3.9-4.5] | 4.2 [3.8-4.6] | 0,003 |
| RBC count (*10^12/1) | 4.6 [4.2-5] | 4.5 [4.1-4.9] | 4.2 [3.8-4.7] | <0.001 |
| Sodium (mmol/L) | 140 [138-142] | 140 [137-142] | 139 [136-141] | <0.001 |
| Triglycerides (mmol/L) | 1.3 [1-1.7] | 1.3 [0.9-1.8] | 1.1 [0.9-1.5] | <0.001 |
| WBC (*10^9/1 | 7.7 [6.6-9.2] | 8 [6.5-9.8] | 7.7 [6.3-9.7] | 0,529 |
| Hematocrit (%) | 41.3±4.9 | 40.2±5.2 | 38.2±5.4 | <0.001 |

| **Biomarkers** | | | | |
|---|---|---|---|---|
| IL-6 (pg/ml) | 3.7 [2-6.8] | 5.1 [2.8-8.9] | 8.2 [4.5-16.5] | <0.001 |
| CRP (ng/ml) | 11872.9 [4810.7-25356.7] | 12266.6 [5506.4-25955.3] | 16350.1 [7718.9-29386.1] | 0,004 |
| D-Dimer (ng/ml) | 101.9 [101.9-101.9] | 101.9 [101.9-146.5] | 101.9 [101.9-194.3] | 0,032 |
| PIGR (ng/ml) | 71.5 [44-106.5] | 123.6 [89.8-177.3] | 216.3 [153.8-311.2] | <0.001 |
| Pentraxin-3 (ng/ml) | 1.3 [0.8-2.1] | 2 [1.4-3.1] | 3.3 [2.1-5.2] | <0.001 |
| GDF-15 (ng/ml) | 2.6 [2.2-3.1] | 3.4 [3-4] | 4.4 [3.8-5.3] | <0.001 |
| RAGE (ng/ml) | 2 [1.4-2.7] | 2.9 [2.1-4] | 3.8 [2.8-5] | <0.001 |
| TNF-R1a (ng/ml) | 0.6 [0.3-0.8] | 1 [0.8-1.4] | 1.9 [1.3-2.7] | <0.001 |
| PCT (pg/ml) | 6.2 [3.7-16.1] | 16 [8.2-31.6] | 36.6 [18.9-82.2] | <0.001 |
| Myeloperoxidase (ng/ml) | 24.3 [20.6-30.2] | 28.4 [24.1-34.8] | 31.1 [26.6-38.9] | <0.001 |
| Syndecan-1 (ng/ml) | 1.2 [0.6-2.1] | 2.2 [1.4-3.5] | 3.9 [2.5-6.1] | <0.001 |
| Periostin (ng/ml) | 3.9 [2.3-6.5] | 6.2 [4-9.5] | 9.2 [5.9-14.9] | <0.001 |
| Galectin-3 (ng/ml) | 17.6 [13.2-24.7] | 20.3 [15.4-27] | 26.8 [19.1-37.7] | <0.001 |
| Osteopontin (ng/ml) | 176.3 [147.2-206.4] | 215.4 [187.2-247] | 260.2 [227.7-298.8] | <0.001 |
| ET1 (pg/ml) | 4.4 [3.5-5.9] | 5.3 [4-6.7] | 6.6 [5.1-9.1] | <0.001 |
| BNP (pg/ml) | 515 [219.9-957.5] | 598.3 [260.7-1162] | 1032 [639-1641.5] | 0,004 |
| NT-proCNP (pg/ml) | 5.2 [5.2-5.2] | 5.3 [5.2-11.2] | 14.7 [6.2-27.4] | <0.001 |
| ST-2 (ng/ml) | 4.2 [2.3-9.1] | 8.3 [4.5-15.6] | 20.4 [10.1-36.1] | <0.001 |
| VEGFR (ng/ml) | 0.1 [0.1-0.1] | 0.1 [0.1-0.2] | 0.2 [0.1-0.4] | <0.001 |
| Angiogenin(ng/mL) | 6202.1 [4115.9-9654.5] | 4484.4 [3193-6378.8] | 3672 [2629-5151.6] | <0.001 |
| Mesothelin (ng/ml) | 47.7 [42.4-53.1] | 53.7 [48.7-59.5] | 60.4 [54.2-68.8] | <0.001 |
| Neuropilin SAND (ng/ml) | 17 [13.5-20.8] | 21.3 [17.5-25.5] | 26.6 [21.4-31.6] | <0.001 |
| Troy (pg/ml) | 0.1 [0.1-0.2] | 0.3 [0.2-0.4] | 0.4 [0.3-0.7] | <0.001 |
| LTBR (ng/ml) | 0.1 [0.1-0.1] | 0.2 [0.1-0.2] | 0.2 [0.2-0.3] | <0.001 |
| ESAM (ng/ml) | 57.1 [51.6-62.2] | 64.2 [59.2-69.1] | 71 [65.8-78] | <0.001 |
| NGAL(ng/ml) | 45.6 [30.7-72.4] | 59 [37.5-87.6] | 82.7 [51-135.4] | <0.001 |
| PSAP-B(ng/ml) | 25.5 [17.2-33] | 31.6 [24.4-37.4] | 37 [29.4-43.1] | <0.001 |
| WAP4C(ng/ml) | 0.7 [0.5-1.2] | 1.4 [0.9-2.3] | 3.5 [2-6.2] | <0.001 |
| cTnI(pg/ml) | 9.6 [5-23.5] | 12.6 [7.2-27] | 16.5 [9.5-35.9] | 0,098 |

### Example 8

### (subgroup analysis PROTECT)

Table 13 shows the data of the PROTECT trial on the association between bio-ADM levels and clinical congestion by left ventricular ejection fraction (LVEF) status. As the ejection fraction based classification is the most relevant phenotyping used in heart failure currently, the analysis was performed in HFrEF vs. HFpEF subgroups. The association between bio-ADM levels and clinical congestion score is comparable in the two subgroups.

**Table 13: Association between baseline bio-ADM levels (log-transformed) and clinical congestion score by ejection fraction status in PROTECT**

| **Outcome** | **Unadjusted OR (95% CI)** | | | **Adjusted OR (95% CI)** | | |
|---|---|---|---|---|---|---|
| | HFrEF | HFpEF | P-interaction* | HFrEF | HFpEF | P-interaction* |
| Clinical congestion at baseline | 1.85 [1.50-2.30] | 1.73 [1.10-2.86] | 0.670 | 1.55 [1.25-1.95] | 1.83 [1.08-3.33] | 0.540 |
| Significant residual congestion by day 7 | 2.91 [2.18-3.96] | 2.86 [1.60-5.68] | 0.450 | 1.90 [1.34-2.77] | 2.10 [0.60-8.74] | 0.802 |

| | | | | | | |
|---|---|---|---|---|---|---|
| 1=adjusted for BMI, serum albumin and past HF hospitalization for outcome 1 2= adjusted for baseline variables including orthopnea, JVP, peripheral edema, history of PCI, pacemaker, ACEI/ARB use, BMI, diastolic blood pressure, blood urea nitrogen, hematocrit and BNP for outcome 2 *interaction tested between log-transformed bio-ADM and left ventricular ejection fraction (LVEF) which was included as a continuous variable | | | | | | |

Heart failure with reduced ejection fraction (HFrEF) was defined as LVEF<40% and heart failure with preserved ejection fraction (HFpEF) as LVEF ≥50%

LVEF data was only available in 763 patients (out of the 1572 patients with available baseline bio-ADM measurements); out of these 545 had HFrEF while 102 had HFpEF.

### Example 9

Serial measurements (monitoring) of bio-ADM in patients with congestion (PROTECT study)

Bio-ADM was measured from plasma collected during follow-up evaluation in hospitalized AHF patients included in the PROTECT trial (all available samples on day 2 and day 7) to monitor the bio-ADM levels with respect to congestion status. Median bio-ADM Levels for all patients at different time points are summarized in table 14. Bio-ADM levels were analyzed over time by treatment success at day 7 (defined based on congestion status by day 7 utilizing the composite congestion score [CCS] as explained above).

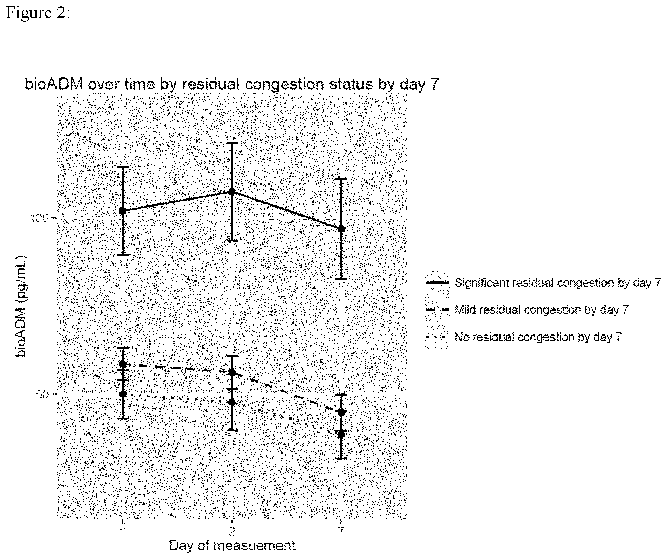

Figure 2 shows that patients with significant residual congestion by day 7 have high baseline levels of bio-ADM and these high levels are sustained after the course of 7 days of therapy, while declining levels compared to baseline are observed by day 7 for patients with only mild or no congestion by day 7.

| **Table 14:** Summary of bio-ADM levels for serial measurements Bio-ADM levels in pg/mL (median [interquartile range]) | | |
|---|---|---|
| Day 1 (N=1562) | Day 2 (N=1466) | Day 7 (N=1454) |
| 44.1 [25.9-82.7] | 43.9 [25.8-77.3] | 34.3 [21.6-61.8] |

### Example 10

### Comparative analysis of predictive value of bio-ADM and MR-proADM for clinically assessed congestion

### a) PROTECT-study

In table 15, a comparative analysis of bio-ADM and MR-proADM in terms of prediction of severity of clinical congestion at baseline and presence of significant residual congestion by day 7 in the PROTECT trial is presented. Clinical congestion was assessed with the composite congestion score (CCS) as previously described. Severity of baseline clinical congestion was graded as mild/ moderate if CCS was <6 and severe if CCS was ≥6. Then unadjusted and adjusted associations between bio-ADM, MR-proADM levels and severity of clinical congestion at baseline was assessed in univariate and multivariate binary logistic regression models. A baseline multivariable model including body mass index, serum albumin, total cholesterol, BNP, history of atrial fibrillation and past HF hospitalization was identified for multivariable analysis after running a backward selection on a model that included variables univariably associated with severity of clinical congestion at baseline at a level of significance of 20.0%. The area under the curve (AUC) was calculated for bio-ADM and MR-proADM to quantify and compare the discriminatory value of the two biomarkers between mild/ moderate and severe clinical congestion.

For residual congestion, CCS evaluated at day 7 was utilized. Significant residual congestion was considered present if CCS by day 7 was ≥3. Again, unadjusted and adjusted associations between bio-ADM, MR-proADM levels and the presence of significant residual congestion by day 7 was assessed in univariate and multivariate binary logistic regression models. A baseline model including orthopnea, jugular venous pressure, peripheral edema, history of percutaneous intervention, pacemaker, ACEI/ARB use, BMI, diastolic blood pressure, blood urea nitrogen, hematocrit and BNP was identified for multivariable analysis after running a backward selection on a model that included variables univariably associated with the presence of significant residual congestion by day 7 at a level of significance of 20.0%. The AUC was calculated to quantify and compare the discriminatory value of the biomarkers for the presence of significant residual congestion.

**Table 15: Comparative analysis of baseline bio-ADM and MR-proADM levels in predicting severity of congestion at baseline and presence of significant residual congestion by day 7 in PROTECT**

| **Severity of clinically assessed congestion at baseline** | | | | | |
|---|---|---|---|---|---|
| Biomarker | Unadjusted | | | Adjusted | |
| | OR [95% CI] | P-value | AUC [95% CI] | OR [95% CI] | P-value |
| bio-ADM (Ln-transformed) | 1.76 [1.56-1.99] | <0.001 | 0.66 [0.63-0.68] | 1.44 [1.25-1.65] | <0.001 |
| MR-proADM | 1.27 [1.14-1.41] | <0.001 | 0.57 [0.54-0.60] | 1.06 [0.93-1.20] | 0.363 |

| **Presence of significant residual congestion by day** 7 | | | | | |
|---|---|---|---|---|---|
| | Unadjusted | | | Adjusted | |
| | OR [95% CI] | P-value | AUC [95% CI] | OR [95% CI] | P-value |
| bio-ADM (Ln-transformed) | 1.87 [1.62-2.17] | <0.001 | 0.66 [0.63-0.70] | 1.28 [1.06-1.54] | 0.009 |
| MR-proADM | 1.36 [1.20-1.54] | <0.001 | 0.59 [0.55-0.62] | 0.92 [0.76-1.10] | 0.362 |

| | | | | | |
|---|---|---|---|---|---|
| *Odds ratios should be interpreted per standard deviation of Ln-transformed bio-ADM and MR-proADM (non-transformed) | | | | | |

### b) BIOSTAT-study

In table 16, association between bio-ADM, MR-proADM levels and the severity of baseline clinical congestion in BIOSTAT is presented. Clinical congestion was evaluated utilizing a slightly different CCS (compared to the one utilized in PROTECT) but a similar statistical approach was utilized for analysis. CCS was calculated by using edema, orthopnea and jugular venous pressure, but the score only ranged from 0-5 as presented in the following table:

| **Parameter** | **0** | **1** | **2** | **3** |
|---|---|---|---|---|
| Peripheral edema | 0 | Ankle | Below knee | Above Knee |
| Orthopnea | Absent | Present | - | - |
| JVP | Not elevated | Elevated | - | - |

Baseline severity of congestion was defined as no/ mild if CCS was <2 (N=709) and significant if CCS was ≥2 (N=635). A binary logistic regression model was utilized to assess unadjusted and adjusted associations. The same baseline model identified in PROTECT (including BMI, serum albumin, total cholesterol, BNP, history of atrial fibrillation and past HF hospitalization) was utilized for multivariable adjustment.

**Table 16: Comparative analysis of baseline bio-ADM and MR-proADM levels in predicting severity of congestion at baseline in BIOSTAT**

| **Severity of clinically assessed congestion at baseline** | | | | | |
|---|---|---|---|---|---|
| Biomarker | Unadjusted | | | Adjusted | |
| | OR [95% CI] | P-value | AUC [95% CI] | OR [95% CI] | P-value |
| bio-ADM, (Ln-transformed) | 3.17 [2.72-3.73] | <0.001 | 0.76 [0.73-0.78] | 2.20 [1.78-2.75] | <0.001 |
| MR-proADM | 2.62 [2.18-3.17] | <0.001 | 0.69 [0.0.67-0.72] | 1.82 [1.42-2.38] | <0.001 |

### Example 11

### a) GREAT-study

### Study population

Three cohorts of unselected AHF patients (n=1075) presenting with acute dyspnea to the emergency department of the participating university hospitals in three countries (United Kingdom, France and Switzerland) were recruited. AHF was defined according to the guidelines of the European Society of Cardiology as progressive worsening or new onset of shortness of breath along with clinical signs of pulmonary or peripheral edema and elevated jugular venous pressure requiring intensification of diuretic and/or vasodilator therapy. Inclusion was independent of renal function, although patients with terminal renal failure on established renal replacement therapy were excluded. These studies complied with the declaration of Helsinki and ethics approval granted from respective research ethics committees. All patients provided written informed consent.

### Plasma sampling

Following signed informed consent, venous blood was withdrawn from recumbent patients and collected in pre-chilled tubes containing EDTA as anticoagulant. The interval for obtaining this admission sample was up to 4 hours (Paris), 1 hour (Basel) and 12 hours (Leicester). Plasma was stored at -80°C until analysis in a single batch.

### Results

Patients characteristics are summarized in table 17, and biomarker distribution is summarized in table 18. During the first year of follow up, 299 died. One of the cohorts (Paris) did not have data on cause of re-hospitalization and were excluded for that endpoint. The remaining centers had follow up data for n=861 patients, of which 345 died or were re-hospitalized for heart failure (HF) within one year.

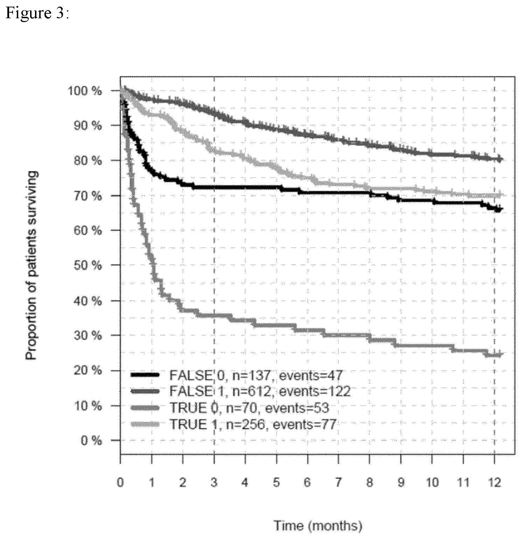

Bio-ADM was associated with 1-year mortality (p<0.0001) and 1-year mortality or re-hospitalization for HF (p<0.0001), both univariate and multivariable (both p<0.0001). Notably, there was an interaction of bio-ADM and treatment with diuretics given on discharge (p=0.0001, table 19 and 20 and figure 3). The standardized HR (standardized for one standard deviation (SD) of log₁₀ transformed bioADM) was 1.7 (p<0.0001) for bio-ADM and 0.61 (p<0.0001) for the interaction term, with diuretic treatment.

**Table 17: Patients characteristics of patients included in analysis.**

| **Admission characteristics** | |
|---|---|
| Number | 1075 |
| Male | 643 (59.8%) |
| Previous HF | 361 (33.5%) |
| Previous IHD | 318 (29.6%) |
| Previous Hypertension | 597 (55.5%) |
| Diabetes | 339 (31.5%) |
| Urea (mM) | 8.8 [2.3 - 46.6] |
| eGFR ml/min/1.73m² | 53.3 [5 - 111] |

**Table 18: Biomarker distribution summary for patients included in analysis at admission and discharge.**

| | NT-proBNP | bio-ADM |
|---|---|---|
| Admission | 2165 | 49.6 |
| | [0.3 - 15902] | [10.5 - 1772.8] |
| Discharge | 1617 | 44.5 |
| | [0.3 - 16233] | [9.5 - 734.2] |

**Table 19: Results of multivariable cox regression analysis for endpoint death one year after admission, including interaction term for treatment with diuretics at discharge and bio-ADM.**

| | | *χ*² | *d.f.* | *P* |
|---|---|---|---|---|
| **Bio-ADM** (Factor+Higher Order Factors) | | 87.71 | 2 | < 0.0001 |
| | *All Interactions* | 13.08 | 1 | 0.0003 |
| D.Diuretic (Factor+Higher Order Factors) | | 96.29 | 2 | < 0.0001 |
| | *All Interactions* | 13.08 | 1 | 0.0003 |
| **Bio-ADM** × D.Diuretic (Factor+Higher Order Factors) | | 13.08 | 1 | 0.0003 |
| **TOTAL** | | 195.65 | 3 | < 0.0001 |

**Table 20: Results of multivariable cox regression analysis for endpoint death or re-hospitalization for heart failure (HF) one year after admission, including interaction term for treatment with diuretics at discharge and bio-ADM.**

| | | χ² | *d.f.* | *P* |
|---|---|---|---|---|
| **Bio-ADM** (Factor+Higher Order Factors) | | 60.19 | 2 | < 0.0001 |
| | *All Interactions* | 15.69 | 1 | 0.0001 |
| D.Diuretic (Factor+Higher Order Factors) | | 82.86 | 2 | < 0.0001 |
| | *All Interactions* | 15.69 | 1 | 0.0001 |
| **Bio-ADM** × D.Diuretic (Factor+Higher Order Factors) | | 15.69 | 1 | 0.0001 |
| **TOTAl.** | | 142.37 | 3 | < 0.0001 |

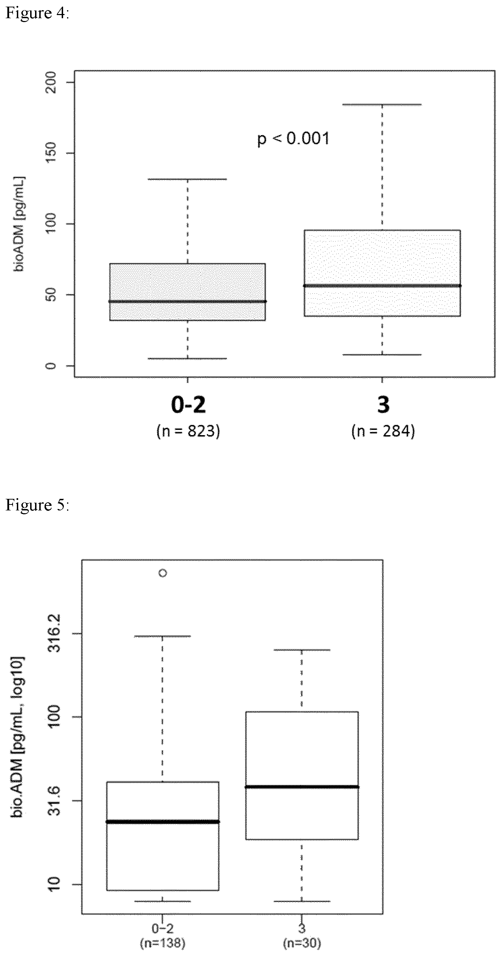

Bio-ADM was also associated with clinical congestion. Data was available from centers Leicester and Paris (n=1107). The clinical congestion score combines presence of peripheral edema, orthopnea and jugular venous pressure >6 cm (each adding a point to the score, see table 21). Bio-ADM was elevated in patients with a clinical congestion score (CCS) of 3 (n=284, p<0.001, figure 4).

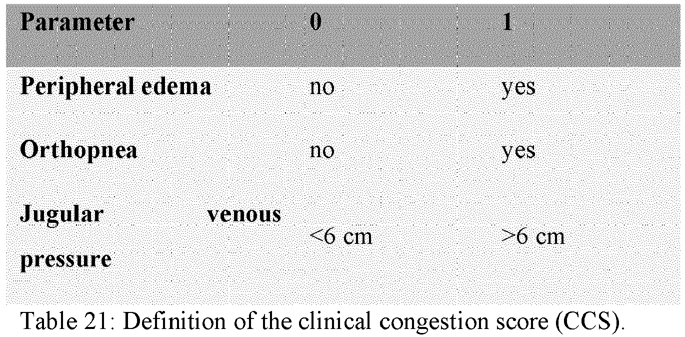

| | |
|---|---|
| CCS = 0 | no congestion |
| CCS = 1-2 | mild congestion |
| CCS = 3 | significant congestion |

### Example 12

### DiSomma-study

### Study population

This was a prospective, observational trial conducted in intensive care unit. We enrolled patients admitted for AHF from the emergency department (ED) of Sant' Andrea hospital in Rome. Clinical and laboratory data including Clinical Congestion Score (CCS: peripheral edema, jugular vein distension and orthopnea), diuretic treatment and bio-ADM values were collected at arrival and patients were followed until hospital discharge. The study was approved by the ethical committee of Sant' Andrea Hospital in Rome. All patients provided written informed consent.

### Plasma sampling

Plasma samples for ADM measurement were obtained on admission.

### Results

209 patients with a final diagnosis of AHF were recruited. The clinical congestion score was available in 168 patients. Patient characteristics are summarized in table 17. 22.6%, 38.1%, 21.4% and 17.9% of patients presented with a CCS of 0, 1, 2 and 3, respectively. Compared to patients with a CCS between 0-2, bio-ADM resulted higher (p=0.01) in patients with a CCS of 3 and in patients with vena cava index >1 (p=0.05), suggesting a potential role of bio-ADM in the detection of congestion, that is considered the main cause of death and re-hospitalization for heart failure patients. Moreover, higher levels of bio-ADM were linked to increase in furosemide treatment and to higher rate of in-hospital mortality, probably related to congestion amount (p=0.002 and p<0.001, respectively).

**Table 22: Patient characteristics.**

| **Variable** | | **all (n=168)** |
|---|---|---|
| **Demographics** | | |
| | Gender - male | 45% |
| | Age - median [IQR) | 80 [73-86) |

| **concomitant diseases** | | |
|---|---|---|
| | Heart failure - yes | 39% |
| | Hypertension - yes | 74% |
| | Diabetes - yes | 36% |
| | Renal dysfunction - yes | 24% |

| **other** | | |
|---|---|---|
| | Creatinine (mg/dL) - median [IQR) | 1.1 [0.9-3.3) |
| | Final diagnosis - pneumonia | 17% |
| | Final diagnosis - AHF | 82% |
| | Intra-hospital mortality | 10% |

Bio-ADM was also associated with clinical congestion. The clinical congestion score combines presence of peripheral edema, orthopnea and jugular venous pressure >6 cm (each adding a point to the score). BioADM was elevated in patients with a clinical congestion score (CCS) of 3 (n=30, p<0.01, figure 5).

## Claims

1. A method for
a) diagnosing congestion or assessing or monitoring the extent of congestion in a subject or,
b) predicting or determining or monitoring need of therapy or intervention of congestion or predicting or determining or monitoring the success of a therapy or intervention of congestion or guiding a therapy or intervention of congestion in a subject or,
c) predicting decongestion or residual congestion after therapy or intervention of congestion in a subject or,
d) assessing decongestion or residual congestion after therapy or intervention of congestion in a subject or,
e) assessing the decision on hospital discharge of a subject,
wherein said subject is subject having acute heart failure that is either new-onset AHF or acute decompensated HF or acute decompensated chronic HF or wherein said subject is a subject having chronic heart failure with worsening signs/symptoms of chronic heart failure and
wherein Pro-Adrenomedullin or fragments thereof of at least 5 amino acids is used as a marker for congestion and
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH2 according to SEQ ID No.: 4 or ADM-Gly according to SEQ ID No.: 5 or CT-proADM according to SEQ ID No.: 6
wherein said subject is stratified into non-responder and/or responder, and/or bad responder to therapy or intervention of congestion.

2. A method of claim 1 comprising the steps:
• Determining the level of Pro-Adrenomedullin or fragments thereof of at least 5 amino acids in a bodily fluid obtained from said subject; and
a) Correlating said level of Pro-Adrenomedullin or fragments thereof with the extent of congestion in said subject or diagnosing congestion, wherein an elevated level above a certain threshold is indicative of congestion or the extent of congestion or,
b) Correlating said level ofPro-Adrenomedullin or fragments thereof with success of a therapy or intervention of congestion in said subject, wherein a level below a certain threshold is predictive for a success of therapy or intervention of congestion, and wherein a level above a certain threshold is indicative for need of therapy or intervention of congestion or,
c) Correlating said level of Pro-Adrenomedullin or fragments thereof with a prediction of decongestion or residual congestion after therapy or intervention of congestion, wherein an elevated level above a certain threshold predicts residual congestion after therapy or intervention of congestion whereas a level below a certain threshold predicts decongestion after therapy or intervention of congestion, or
d) Correlating said level of Pro-Adrenomedullin or fragments thereof with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
e) Correlating said level of Pro-Adrenomedullin or fragments thereof with the assessment of the decision on hospital discharge, wherein an elevated level above a certain threshold means that the subject shall not be discharged and wherein a level below a certain threshold means that the subject may be discharged,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂

3. A method according to claim 1 comprising the steps:
• Determining the level of immunoreactive analyte by using at least one binder that binds to a region within the amino acid sequence of Pro-Adrenomedullin or fragments thereof of at least 5 amino acids in a bodily fluid obtained from said subject; and
a) Correlating said level of immunoreactive analyte with the extent of congestion in said subject or diagnosing congestion, wherein an elevated level above a certain threshold is indicative of congestion or the extent of congestion, or
b) Correlating said level of immunoreactive analyte with success of a therapy or intervention of congestion in said subject, wherein a level below a certain threshold is predictive for a success of therapy or intervention of congestion, and wherein a level above a certain threshold is indicative for need of therapy or intervention of congestion, or
c) Correlating said level of immunoreactive analyte with a prediction of decongestion or residual congestion after therapy or intervention of congestion, wherein an elevated level above a certain threshold predicts residual congestion after therapy or intervention of congestion whereas a level below a certain threshold predicts decongestion after therapy, or
d) Correlating said level of immunoreactive analyte with decongestion or residual congestion after therapy or intervention of congestion, wherein an elevated level above a certain threshold indicates residual congestion after therapy or intervention of congestion whereas a level below a certain threshold indicates decongestion after therapy or intervention of congestion, or
e) Correlating said level of immunoreactive analyte with the assessment of the decision on hospital discharge wherein an elevated level above a certain threshold means that the subject shall not be discharged and wherein a level below a certain threshold means that the subject may be discharged,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6.

4. A method according to any of claims 1 to 3, wherein said extent of congestion is expressed as congestion score, in particular a clinical congestion score.

5. A method according to any of claims 1 to 4 wherein said fragment may be selected from MR-proADM according to SEQ ID No.: 3 or mature ADM-NH₂ according to SEQ ID No.: 4.

6. A method according to any of claims 1 to 5, wherein the level of Pro-Adrenomedullin or fragments thereof of at least 5 amino acids is determined by using a binder to Pro-Adrenomedullin or fragments thereof of at least 5 amino acids.

7. A method according to claim 6, wherein the binder is selected from the group comprising an antibody, an antibody fragment or a non-Ig-Scaffold binding to Pro-Adrenomedullin or fragments thereof of at least 5 amino acids.

8. A method according to any of the preceding claims, wherein said threshold is within a threshold range that is a threshold range for plasma mature ADM-NH₂ according to SEQ ID No.: 4 between 50 and 100 pg/ml and for plasma MR-proADM between 0.5 and 1.5 nmol/L, and for plasma CT-proADM between 85 and 350 pmol/L.

9. A method according to any of claims 1 to 8, wherein said determination of Pro-Adrenomedullin or fragments thereof of at least 5 amino acids is performed more than once in one patient.

10. A method according to any of claims 1 to 9, wherein the sample is taken at admission to a hospital or discharge of hospital.

11. A method according to any of claims 1 to 10, wherein said monitoring is performed in order to evaluate the response of said subject to preventive and/or therapeutic measures taken.

12. A method according to any of claims 1 to 11, wherein said level of Pro-Adrenomedullin or fragments thereof or said level of immunoreactive analyte is used for guidance of therapy or intervention of congestion, wherein therapy or intervention is indicated if said level of Pro-Adrenomedullin or fragments is above a certain threshold and wherein-, therapy or intervention of congestion is not indicated if said level of Pro-Adrenomedullin or fragments is below a certain threshold.

13. A method according to any of claims 1 to 12, wherein said level of Pro-Adrenomedullin or fragments thereof or said level of immunoreactive analyte is used for determining the need of therapy or intervention of congestion.

14. A method according to any of claims 1 to 13, wherein said intervention or therapy is selected from the group comprising administration of diuretics, administration of inotropes, administration of vasodilators, ultrafiltration.
